# EUROPÄISCHE PATENTSCHRIFT

(11) **EP 1 087 948 B1**
(45) Veröffentlichungstag und Bekanntmachung des Hinweises auf die Patenterteilung: **03.08.2005**
(21) Anmeldenummer: 99927843.5
(22) Anmeldetag: 02.06.1999
(51) Int. Cl.: C07D 251/18, C07D 407/12, C07D 413/12, C07D 417/12, C07D 403/12, A01N 43/68, C07C 279/26

(54) **2,4-DIAMINO-1,3,5-TRIAZINE, VERFAHREN ZUR HERSTELLUNG UND VERWENDUNG ALS HERBIZIDE UND PFLANZENWACHSTUMSREGULATOREN**
2,4-DIAMINO-1,3,5-TRIAZINE, METHOD FOR THE PRODUCTION AND USE OF THE SAME AS HERBICIDES AND PLANT GROWTH REGULATORS
2,4-DIAMINO-1,3,5-TRIAZINES, LEUR PROCEDE DE PRODUCTION ET LEUR UTILISATION COMME HERBICIDES ET REGULATEURS DE LA CROISSANCE VEGETALE

(30) Priorität: 16.06.1998 DE 19826670
(43) Veröffentlichungstag der Anmeldung: 04.04.2001
(73) Patentinhaber: Bayer CropScience GmbH, 65929 Frankfurt/Main (DE)
(72) Erfinder: GIENCKE, Wolfgang, D-65719 Hofheim (DE); MINN, Klemens, D-65795 Hattersheim (DE); WILLMS, Lothar, D-65719 Hofheim (DE); AULER, Thomas, D-65812 Bad Soden (DE); BIERINGER, Hermann, D-65817 Eppstein (DE); ROSINGER, Christopher, D-65719 Hofheim (DE)
(86) Internationale Anmeldenummer: PCT/EP1999/003817
(87) Internationale Veröffentlichungsnummer: WO 1999/065882

(56) Entgegenhaltungen:
- BE-A- 612 529
- DE-A- 19 522 137
- DE-A- 19 531 084
- DE-A- 19 641 691
- DE-A- 19 641 694
- SHAPIRO ET AL: "Guanamine diuretics" JOURNAL OF THE AMERICAN CHEMICAL SOCIETY., Bd. 79, 1957, Seiten 5064-5071, XP002110860 AMERICAN CHEMICAL SOCIETY, WASHINGTON, DC., US ISSN: 0002-7863
- SHAPIRO ET AL: "Hypoglycemic agents" JOURNAL OF THE AMERICAN CHEMICAL SOCIETY., Bd. 81, 1959, Seiten 3728-3736, XP002110861 AMERICAN CHEMICAL SOCIETY, WASHINGTON, DC., US ISSN: 0002-7863
- CHEMICAL ABSTRACTS, vol. 56, no. 6, 19. März 1962 (1962-03-19) Columbus, Ohio, US; abstract no. 6466a, WEINBERG ET AL: "The antimicrobial activity of N1,N2-substituted biguanides" XP002110863 -& DATABASE CAOLD [Online] CHEMICAL ABSTRACTS SERVICE, COLUMBUS, OHIO, US CA56:6466a, XP002110875
- CHEMICAL ABSTRACTS, vol. 59, no. 9, 28. Oktober 1963 (1963-10-28) Columbus, Ohio, US; abstract no. 9872d, PAUL ET AL: "Synthesis of biguanides as potential hypoglemic agents" XP002110864 -& DATABASE CAOLD [Online] CHEMICAL ABSTRACTS SERVICE, COLUMBUS, OHIO, US CA59:9872d, XP002110876
- DATABASE CROSSFIRE [Online] Beilstein, XP002110865 & CHEMISCHE BERICHTE., Bd. 22, 1889, Seite 1409 VERLAG CHEMIE GMBH. WEINHEIM., DE ISSN: 0009-2940
- DATABASE CROSSFIRE [Online] Beilstein XP002110866 & ARZNEIM. FORSCH.,1978, Seiten 1561-1564,
- GOOSSEN, LUKAS J. ET AL: "Catalytic asymmetric aminohydroxylation with amino-substituted heterocycles as nitrogen sources" ANGEW. CHEM., INT. ED. (1999), 38(8), 1080-1083, XP002110862

## Beschreibung

Die Erfindung betrifft das technische Gebiet der Pflanzenschutzmittel, wie Herbizide und Pflanzenwachtumsregulatoren, insbesondere der Herbizide zur selektiven Bekämpfung von Schadpflanzen in Nutzpflanzenkulturen.

Es ist bekannt, daß in 6-Stellung substituierte 2-Amino-4-(N-Phenylalkyl-amino)-1,3,5-triazine, die noch weiter substituiert sein können, herbizide und pflanzenwachstumsregulierende Eigenschaften besitzen; vgl. WO97/08156 und dort zitierte Literatur und WO98/15537und dort zitierte Literatur; vgl. teilweise auch WO 97/00254 und dort zitierte Literatur).

Die bekannten Wirkstoffe weisen bei ihrer Anwendung teilweise Nachteile auf, sei es unzureichende herbizide Wirkung gegen Schadpflanzen, zu geringes Spektrum der Schadpflanzen, das mit einem Wirkstoff bekämpft werden kann, oder zu geringe Selektivität in Nutzpflanzenkulturen. Andere Wirkstoffe lassen sich wegen schwer zugänglicher Vorprodukte und Reagenzien im industriellen Maßstab nicht wirtschaftlich herstellen oder besitzen nur unzureichende chemische Stabilitäten.

Aufgabe der Erfindung ist es, alternative Wirkstoffe vom Typ der 2,4-Diamino-1,3,5-Triazine bereitzustellen, die gegebenenfalls mit Vorteilen als Herbizide oder Pflanzenwachstumsregulatoren eingesetzt werden können.

Gegenstand der vorliegenden Erfindung sind Verbindungen der Formel (I) und deren Salze, 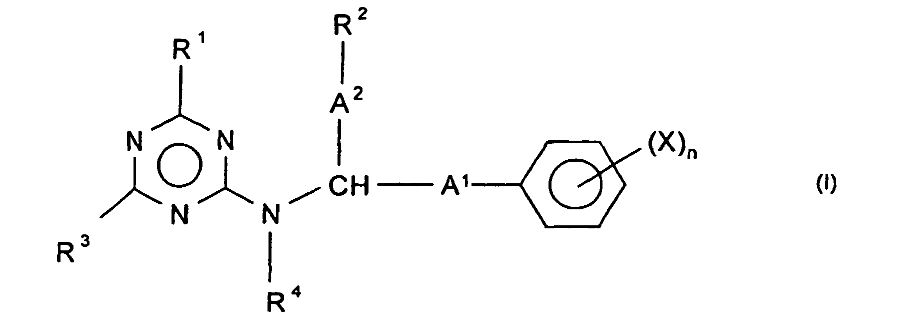 worin
- R¹: Phenyl, das unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist,
und inklusive Substituenten vorzugsweise 6 bis 30 C-Atome aufweist,
oder
(C₃-C₉)Cycloalkyl, das unsubstituiert oder durch einen oder mehrere Reste aus der Gruppe Halogen, Hydroxy, Amino, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist, und inklusive Substituenten vorzugsweise 3 bis 30 C-Atome aufweist,
oder
Heterocyclyl, das unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist, und inklusive Substituenten vorzugsweise 2 bis 30 C-Atome aufweist,
oder
(C₁-C₄)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Alkylsulfinyl, (C₁-C₄)Alkylsulfonyl, (C₁-C₄)Haloalkylsulfinyl, (C₁-C₄)Haloalkylsulfonyl und (C₃-C₆)Cycloalkyl, das unsubstituiert oder oder durch einen oder mehrere Reste aus der Gruppe Halogen, Hydroxy, Amino, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist, und Phenyl und Heterocyclyl, wobei jeder der zwei letztgenannten Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist, und Reste der Formeln R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- und R'R"N-C(=Z')-NR"'-,
worin R', R" und R"' jeweils unabhängig voneinander (C₁-C₄)Alkyl, Phenyl, Phenyl-(C₁-C₄)alkyl, (C₃-C₆)Cycloalkyl oder (C₃-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Nitro, Formyl, Cyano Thiocyanato, (C₁-C₄)Alkoxy, (C₁-C₄)Alkylthio, Mono(C₁-C₄)alkylamiro, Di(C₁-C₄)alkylamino, (C₂-C₄)Alkenyl, (C₂-C₄)Alkinyl, (C₃-C₆)Cycloalkyl und im Fall cyclischer Reste auch (C₁-C₄)Alkyl und (C₁-C₄)Haloalkyl substituiert ist, bedeuten und worin Z und Z' unabhängig voneinander jeweils ein Sauerstoff- oder Schwefelatom sind, substituiert ist, und inklusive Substituenten vorzugsweise 1 bis 30 C-Atome aufweist,
- A¹: geradkettiges Alkylen der Formel CH₂CH₂ und
- A²-R²: Cyclopropyl-methyl, 2-Cyclopropyl-ethyl, Cyclobutyl, Cyclobutyl-methyl oder 2-Cyclobutyl-ethyl, oder
- A¹: geradkettiges Alkylen der Formel CH₂CH₂CH₂ und
- A²-R²: Cyclopropyl, Cyclopropyl-methyl, 2-Cyclopropyl-ethyl, Cyclobutyl, Cyclobutylmethyl oder 2-Cyclobutyl-ethyl, und
- R³: Wasserstoff, (C₁-C₄)Alkyl, das unsubstituiert oder durch einen oder mehrere Reste aus der Gruppe Halogen, Hydroxy, Amino, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist, oder Phenyl oder (C₃-C₆)Cycloalkyl, wobei jeder der letztgenannten 2 Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylaminocarbonyl, (C₁-C₄)Alkylsulfonyl und (C₁-C₄)Haloalkylsulfonyl substituiert ist, oder einen Rest der Formel N(B¹-D¹)(B²-D²),
wobei R³ inklusive Substituenten vorzugsweise bis zu 20 C-Atome aufweist,
- R⁴: einen Rest der Formel -B³-D³, wobei B³ und D³ wie unten definiert sind und R⁴ inklusive Substituenten vorzugsweise bis zu 20 C-Atome aufweist,
- B¹, B² und B³: unabhängig voneinander eine direkte Bindung oder eine divalente Gruppe der Formeln -C(=Z*)-, -C(=Z*)-Z**-, -C(=Z*)-NH- oder -C(=Z*)-NR*-, wobei Z* = O oder S, Z** = O oder S und R* =(C₁-C₄)Alkyl, Phenyl, Phenyl-(C₁-C₄)alkyl, (C₃-C₆)Cycloalkyl oder (C₃-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl, (C₁-C₄)Haloalkylsulfonyl und im Fall cyclischer Reste auch (C₁-C₄)Alkyl und (C₁-C₄)Haloalkyl substituiert ist und inklusive Substituenten vorzugsweise bis zu 20 C-Atome aufweist,
- D¹, D² und D³: unabhängig voneinander Wasserstoff, (C₁-C₆)Alkyl, Phenyl, Phenyl-(C₁-C₄)alkyl, (C₃-C₆)Cycloalkyl oder (C₃-C₆)Cycloalkyl-(C₁-C₆)alkyl, wobei jeder der 5 letztgenannten Reste unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl, (C₁-C₄)Haloalkylsulfonyl und im Fall cyclischer Reste auch (C₁-C₄)Alkyl und (C₁-C₄)Haloalkyl substituiert ist und inklusive Substituenten vorzugsweise bis zu 20 C-Atome aufweist,
- (X)ₙ: n Substituenten X und dabei X unabhängig voneinander Halogen, Hydroxy, Amino, Nitro, Formyl, Carboxy, Cyano, Thiocyanato, Aminocarbonyl oder (C₁-C₄)Alkyl, (C₁-C₄)Alkoxy, (C₁-C₄)Alkylthio, Mono(C₁-C₄)alkylamino, Di(C₁-C₄)alkylamino, (C₂-C₄)Alkenyl, (C₂-C₄)Alkinyl, [(C₁-C₄)Alkyl]carbonyl, [(C₁-C₄)Alkoxy]carbonyl, Mono(C₁-C₄)alkylamino-carbonyl, Di(C₁-C₄)alkylamino-carbonyl, N-(C₁-C₆)Alkanoyl-amino oder N-(C₁-C₄)Alkanoyl-N-(C₁-C₄)alkyl-amino,
wobei jeder der letztgenannten 13 Reste unsubstituiert oder durch einen oder mehrere Reste aus der Gruppe Halogen, Hydroxy, Amino, Cyano, Thiocyanato, (C₁-C₄)Alkoxy, (C₁-C₄)Haloalkoxy, (C₁-C₄)Alkylthio, Mono(C₁-C₄)alkylamino, Di(C₁-C₄)alkylamino, (C₃-C₆)Cycloalkyl, (C₃-C₆)Cycloalkylamino, [(C₁-C₄)Alkyl]carbonyl, [(C₁-C₄)Alkoxy]carbonyl, Aminocarbonyl, Mono(C₁-C₄)alkylaminocarbonyl, Di(C₁-C₄)alkylamino-carbonyl, Phenyl, Phenoxy, Phenylthio, Phenylcarbonyl, Heterocyclyl, Heterocyclyloxy, Heterocyclylthio und Heterocyclylamino,
wobei jeder der letztgenannten 8 Reste unsubstituiert ist oder einen oder mehrere Substituenten aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl und (C₁-C₄)Alkoxycarbonyl aufweist, substituiert ist,
oder (C₃-C₉)Cycloalkyl, Phenyl, Phenoxy, Phenylthio, Phenylcarbonyl, Heterocyclyl, Heterocyclyloxy, Heterocyclylthio oder Heterocyclylamino,
wobei jeder der letztgenannten 9 Reste unsubstituiert oder durch einen oder mehrere Reste aus der Gruppe Halogen, Hydroxy, Amino, Nitro, Formyl, Carboxy, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₆)Cycloalkyl, [(C₁-C₄)Alkyl]carbonyl, [(C₁-C₄)Alkoxy]carbonyl, Aminocarbonyl, Mono(C₁-C₄)alkylamino-carbonyl und Di(C₁-C₄)alkylamino-carbonyl substituiert ist,
oder zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist, und
- n: 0, 1, 2 oder 3, vorzugsweise 0, 1, 2, 3 oder 4, insbesondere 1 oder 2
bedeuten und
Heterocyclyl in den vorstehend genannten Resten unabhängig voneinander jeweils einen heterocyclischen Rest mit 3 bis 7 Ringatomen und 1 bis 3 Heteroatomen aus der Gruppe N, O und S bedeutet.

Wenn nicht näher angegeben, sind divalente Reste, z.B. B¹ = -C(=Z*)-Z**-, so definiert, daß in den zusammengesetzten Gruppen, z. B. - B¹-D¹, diejenige Bindung des divalenten Restes mit der Gruppe D¹ verbunden ist, die in der Formel für den divalenten Rest rechts geschrieben ist, d. h. - B¹-D¹ ist eine Gruppe der Formel -C(=Z*)-Z**-D¹; entsprechendes gilt für analoge divalente Reste.

Die Verbindungen der Formel (I) können durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z.B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, daß bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quatemäre) Ammoniumsalze.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Cycloalkyl bedeutet ein carbocyclisches, gesättigtes Ringsystem mit vorzugsweise 3-8 C-Atomen, z.B. Cyclopropyl, Cyclobutyl, Cyclopentyl oder Cyclohexyl. Im Falle von substituiertem Cycloalkyl werden cyclische Systeme mit Substituenten umfaßt, wobei die Substituenten mit einer Doppelbindung am Cycloalkylrest, z. B. eine Alkylidengruppe wie Methyliden, gebunden sind. Im Falle von substituiertem Cycloalkyl werden auch mehrcyclische aliphatische Systeme umfaßt, wie beispielsweise Bicydo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Adamantan-1-yl und Adamantan-2-yl. Cycloalkenyl bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B.

OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch sein; er enthält vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Thiadiazolyl, Oxazolyl, Isoxazolyl, Furyl, Pyrrolyl, Pyrazolyl, Imidazolyl und Triazolyl, oder ist ein partiell oder vollständig hydrierter Rest wie Oxiranyl, Oxetanyl, Oxolanyl (= Tetrahydrofuryl), Oxanyl, Pyrrolidyl, Piperidyl, Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie ein substituierter Alkyl-, Alkenyl-, Alkinyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind, z. B. mit einer Alkylidengruppe wie Methyliden oder Ethyliden substituiert sind.
Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy und Chlor.

Mono- oder disubstituiertes Amino bedeutet einen chemisch stabilen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Monoalkylamino, Dialkylamino, Acylamino, Arylamino, N-Alkyl-N-arylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluor- und -Trichlorphenyl, 2,4-, 3,5-, 2,5- und 2,3-Dichlorphenyl, o-, m- und p-Methoxyphenyl.

Ein Acylrest bedeutet den Rest einer organischen Säure, z.B. den Rest einer Carbonsäure und Reste davon abgeleiteter Säuren wie der Thiocarbonsäure, gegebenenfalls N-substituierten Iminocarbonsäuren oder den Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch ein oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Vor allem aus den Gründen der höheren herbiziden Wirkung, besseren Selektivität und/oder besseren Herstellbarkeit sind erfindungsgemäße Verbindungen der genannten Formel (I) oder deren Salze von besonderem Interesse, worin einzelne Reste eine der bereits genannten oder im folgenden genannten bevorzugten Bedeutungen haben, oder insbesondere solche, worin eine oder mehrere der bereits genannten oder im folgenden genannten bevorzugten Bedeutungen kombiniert auftreten.
- R¹: ist vorzugsweise Phenyl, das unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 6 bis 30 C-Atome, vorzugsweise 6 bis 20 C-Atome, insbesondere 6 bis 15 C-atome aufweist.
- R': ist vorzugsweise auch (C₃-C₉)Cycloalkyl, das unsubstituiert oder durch einen oder mehrere Reste aus der Gruppe Halogen, Hydroxy, Amino, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist und inklusive Substituenten 3 bis 30 C-Atome, vorzugsweise 3 bis 20 C-Atome, insbesondere 3 bis 15 C-atome aufweist.
- R¹: ist vorzugsweise auch Heterocyclyl, das unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 2 bis 30 C-Atome, vorzugsweise 2 bis 20 C-Atome,
insbesondere 2 bis 15 C-atome aufweist.

Dabei und auch in anderen Resten ist Heterocyclyl vorzugsweise ein heterocyclischer Rest mit 3 bis 7, insbesondere 3 bis 6 Ringatomen und einem Heteroatom aus der Gruppe N, O und S, beispielsweise Pyridyl, Thienyl, Furyl, Pyrrolyl, Oxiranyl, Oxetanyl, Oxolanyl (= Tetrahydrofuryl), Oxanyl, Pyrrolidyl, Piperidyl, oder ist ein heterocyclischer Rest mit zwei oder drei Heteroatomen aus der Gruppe Pyrimidinyl, Pyridazinyl, Pyrazinyl, Triazinyl, Thienyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl, Morpholinyl.
- R¹: ist vorzugsweise auch (C₁-C₆)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Alkylsulfinyl, (C₁-C₄)Alkylsulfonyl, (C₁-C₄)Haloalkylsulfinyl, (C₁-C₄)Haloalkylsulfonyl und (C₃-C₆)Cycloalkyl, das unsubstituiert oder oder durch einen oder mehrere Reste aus der Gruppe Halogen, Hydroxy, Amino, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist, und Phenyl und Heterocyclyl, wobei jeder der zwei letztgenannten Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist, und Reste der Formeln R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- und R'R"N-C(=Z')-NR"'-, worin R', R" und R"' jeweils unabhängig voneinander (C₁-C₄)Alkyl, Phenyl, Phenyl-(C₁-C₄)alkyl, (C₃-C₆)Cycloalkyl oder (C₃-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Nitro, Formyl, Cyano, Thiocyanato, (C₁-C₄)Alkoxy, (C₁-C₄)Alkylthio, Mono(C₁-C₄)alkylamino, Di(C₁-C₄)alkylamino, (C₂-C₄)Alkenyl, (C₂-C₄)Alkinyl, (C₃-C₆)Cycloalkyl und im Fall cyclischer Reste auch (C₁-C₄)Alkyl und (C₁-C₄)Haloalkyl substituiert ist, bedeuten und worin Z und Z' unabhängig voneinander jeweils ein Sauerstoff- oder Schwefelatom sind,
substituiert ist und inklusive Substituenten vorzugsweise 1 bis 20 C-Atome, insbesondere 1 bis 15 C-Atome aufweist,
- R¹: ist bevorzugt (C₁-C₄)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, (C₃-C₉)Cycloalkyl, das unsubstituiert oder substituiert ist, und Phenyl, das unsubstituiert oder durch einen oder mehrere Reste aus der Gruppe Halogen, (C₁-C₄)Alkyl und (C₁-C₄)Haloalkyl, (C₁-C₄)Alkoxy, (C₁-C₄)Haloalkoxy, (C₁-C₄)Alkylthio, Amino, Mono- und Di[(C₁-C₄)alkyl]amino, (C₁-C₄)Alkanoylamino, Benzoylamino, Nitro, Cyano, [(C₁-C₄)Alkyl]carbonyl, Formyl, Carbamoyl, Mono- und Di-[(C₁-C₄)alkyl]aminocarbonyl und (C₁-C₄)Alkylsulfonyl substituiert ist, und Heterocyclyl mit 3 bis 6 Ringatomen und 1 bis 3 Heteroringatomen aus der Gruppe N, O und S, wobei der Ring unsubstituiert oder durch einen oder mehrere Reste aus der Gruppe Halogen, (C₁-C₄)Alkyl und Oxo substituiert ist, substituiert ist, oder Phenyl, das unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist und inklusive Substituenten 2 bis 30 C-Atome, vorzugsweise 2 bis 20 C-Atome, insbesondere 2 bis 15 C-atome aufweist.
- R¹: ist weiter bevorzugt (C₁-C₄)Alkyl, (C₁-C₄)Haloalkyl, Benzyl oder [(C₃-C₆)Cycloalkyl]-(C₁-C₂)alkyl, insbesondere (C₁-C₄)Alkyl, (C₁-C₄)Haloalkyl oder [(C₃-C₆)Cycloalkyl]-methyl, vorzugsweise -CH₃, -CH₂F, -CHF₂, -CF₃, -CH₂Cl, -CHCl₂, -CCl₃, -CH₂Br, -CHBr₂, -CH₂CH₃, -CH₂CH₂F, -CF₂CHF₂, -CH₂CH₂Cl, -CH₂CH₂Br -CH(CH₃)₂, -CF(CH₃)₂, -C(CH₃)₂Cl, -CH₂CH₂CH₂F, -CH₂CH₂CH₂Cl oder Cyclopropylmethyl.

Unabhängig von den Resten R¹, R², R⁴, A¹, A² und (X)ₙ und vorzugsweise in Kombination mit bevorzugten Bedeutungen von einem oder mehreren dieser Reste sind folgende Bedeutungen von R³ von besonderem Interesse:
- R³: bedeutet beispielsweise Wasserstoff, (C₁-C₄)Alkyl, das unsubstituiert oder durch einen oder mehrere Reste aus der Gruppe Halogen, Hydroxy, Amino, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist, oder Phenyl oder (C₃-C₆)Cycloalkyl, wobei jeder der letztgenannten 2 Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist, oder einen Rest der Formel N(B¹-D¹)(B²-D²), wobei B¹, B², D¹ und D² wie bereits definiert oder vorzugsweise wie weiter unten definiert sind, insbesondere Amino.

Unabhängig von den Resten R¹ bis R³, A¹, A² und (X)ₙ und vorzugsweise in Kombination mit bevorzugten Bedeutungen von einem oder mehreren dieser Reste sind folgende Bedeutungen von R⁴ von besonderem Interesse:
- R⁴: bedeutet beispielsweise einen Rest der Formel -B³-D³, wobei B³ und D³ vorzugsweise wie weiter unten definiert sind.
- R⁴: ist vorzugsweise Wasserstoff, (C₁-C₄)Alkyl, Phenyl oder (C₃-C₆)Cycloalkyl, wobei jeder der 3 letztgenannten Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl, (C₁-C₄)Haloalkylsulfonyl und im Fall cyclischer Reste auch (C₁-C₄)Alkyl und (C₁-C₄)Haloalkyl substituiert ist, oder
Formyl, [(C₁-C₄)Alkyl]carbonyl, [(C₁-C₄)Alkoxy]carbonyl, Aminocarbonyl, Mono(C₁-C₄)alkylamino-carbonyl oder Di(C₁-C₄)alkylamino-carbonyl; insbesondere Wasserstoff, Methyl, Ethyl, n-Propyl oder Isopropyl; ganz bevorzugt Wasserstoff.

Unabhängig von den Resten R¹ bis R⁴, A² und (X)ₙ und vorzugsweise in Kombination mit bevorzugten Bedeutungen von einem oder mehreren dieser Reste sind folgende Bedeutungen von A¹ von besonderem Interesse:
- A¹: ist vorzugsweise ein Rest der Formel -CH₂CH₂- oder -CH₂CH₂CH₂-, der unsubstituiert ist. Besonders bevorzugt ist A¹ ein Rest der Formel -CH₂CH₂- oder -CH₂CH₂CH₂-, der unsubstituiert ist.

Unabhängig von den Resten R¹ bis R⁴, A¹ und (X)ₙ und vorzugsweise in Kombination mit bevorzugten Bedeutungen von einem oder mehreren dieser Reste sind folgende Bedeutungen von A² von besonderem Interesse:
- A²: ist vorzugsweise eine direkte Bindung oder eine Gruppe der Formel -CH₂- oder -CH₂CH₂-,
- A²: ist besonders bevorzugt eine direkte Bindung oder eine Gruppe der Formel -CH₂- oder -CH₂CH₂-.
- B¹, B² und B³: sind vorzugsweise jeweils unabhängig voneinander eine direkte Bindung oder eine divalente Gruppe der Formeln -C(=Z*)-, -C(=Z*)-Z**-, -C(=Z*)-NH- oder -C(=Z*)-NR*-, wobei Z* = O oder S, Z** = O oder S und R* = (C₁-C₄)Alkyl, Phenyl, Phenyl-(C₁-C₄)alkyl, (C₃-C₆)Cycloalkyl oder (C₃-C₆)Cycloalkyl-(C₁-C₄)alkyl, wobei jeder der 5 letztgenannten Reste unsubstituiert oder substituiert ist, vorzugsweise unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl, (C₁-C₄)Haloalkylsulfonyl und im Fall cyclischer Reste auch (C₁-C₄)Alkyl und (C₁-C₄)Haloalkyl substituiert ist;
weiter bevorzugt sind B¹, B² und B³ unabhängig voneinander eine direkte Bindung oder eine divalente Gruppe der Formeln -C(=Z*)-, -C(=Z*)-Z**-, -C(=Z*)-NH- oder -C(=Z*)-NR*-, wobei Z* = O oder S, Z** = O oder S und R* = (C₁-C₄)Alkyl, Phenyl, Phenyl-(C₁-C₄)alkyl, (C₃-C₆)Cycloalkyl oder (C₃-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Formyl, (C₁-C₄)Alkoxy, (C₁-C₄)Haloalkoxy, (C₁-C₄)Alkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylaminocarbonyl und im Fall cyclischer Reste auch (C₁-C₄)Alkyl und (C₁-C₄)Haloalkyl substituiert ist, sind, insbesondere R* = (C₁-C₄)Alkyl oder (C₃-C₆)Cycloalkyl oder insbesondere R* = Phenyl oder Phenyl-(C₁-C₄)alkyl, wobei jeder der beiden letztgenannten Reste im Phenylteil unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy substituiert ist, ist.
- D¹, D² und D³: bedeuten vorzugsweise unabhängig voneinander Wasserstoff, (C₁-C₆)Alkyl, Phenyl, Phenyl-(C₁-C₄)alkyl, (C₃-C₆)Cycloalkyl oder (C₃-C₆)Cycloalkyl-(C₁-C₆)alkyl, wobei jeder der 5 letztgenannten Reste unsubstituiert oder substituiert ist, vorzugsweise unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl, (C₁-C₄)Haloalkylsulfonyl und im Fall cyclischer Reste auch (C₁-C₄)Alkyl und (C₁-C₄)Haloalkyl substituiert ist.
- Weiter: bevorzugt bedeuten D¹, D² D³ und unabhängig voneinander (C₁-C₄)Alkyl, Phenyl, Phenyl-(C₁-C₄)alkyl, (C₃-C₆)Cycloalkyl oder (C₃-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Formyl, (C₁-C₄)Alkoxy, (C₁-C₄)Haloalkoxy, (C₁-C₄)Alkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylaminocarbonyl und im Fall cyclischer Reste auch (C₁-C₄)Alkyl und (C₁-C₄)Haloalkyl substituiert ist, und bedeuten insbesondere (C₁-C₄)Alkyl oder (C₃-C₆)Cycloalkyl oder Phenyl oder Phenyl-(C₁-C₄)alkyl, wobei jeder der beiden letztgenannten Reste im Phenylteil unsubstituiert oder durch einen oder mehrere Reste aus der Gruppe Halogen, (C₁-C₄)Alkyl, (C₁-C₄)Haloalkyl, (C₁-C₄)Alkoxy oder (C₁-C₄)Haloalkoxy substituiert ist.

Unabhängig von den Resten R¹ bis R⁴, A¹ und A² und vorzugsweise in Kombination mit bevorzugten Bedeutungen von einem oder mehreren dieser Reste sind folgende Bedeutungen von (X)ₙ von besonderem Interesse:
- (X)ₙ: bedeuten n Substituenten X und dabei X vorzugsweise jeweils unabhängig voneinander Halogen, Hydroxy, Amino, Nitro, Formyl, Carboxy, Cyano, Thiocyanato, Aminocarbonyl oder (C₁-C₄)Alkyl, (C₁-C₄)Alkoxy, (C₁-C₄)Alkylthio, Mono(C₁-C₄)alkylamino, Di(C₁-C₄)alkylamino, (C₂-C₄)Alkenyl, (C₂-C₄)Alkinyl, [(C₁-C₄)Alkyl]carbonyl, [(C₁-C₄)Alkoxy]carbonyl, Mono(C₁-C₄)alkylamino-carbonyl, Di(C₁-C₄)alkylamino-carbonyl, N-(C₁-C₆)Alkanoyl-amino oder N-(C₁-C₄)Alkanoyl-N-(C₁-C₄)alkyl-amino,
wobei jeder der letztgenannten 13 Reste unsubstituiert oder substituiert ist, vorzugsweise unsubstituiert oder durch einen oder mehrere Reste aus der Gruppe Halogen, Hydroxy, Amino, Cyano, Thiocyanato, (C₁-C₄)Alkoxy, (C₁-C₄)Haloalkoxy, (C₁-C₄)Alkylthio, Mono(C₁-C₄)alkylamino, Di(C₁-C₄)alkylamino, (C₃-C₆)Cycloalkyl, (C₃-C₆)Cycloalkylamino, [(C₁-C₄)Alkyl]carbonyl, [(C₁-C₄)Alkoxy]carbonyl, Aminocarbonyl, Mono(C₁-C₄)alkylaminocarbonyl, Di(C₁-C₄)alkylamino-carbonyl, Phenyl, Phenoxy, Phenylthio, Phenylcarbonyl, Heterocyclyl, Heterocyclyloxy, Heterocyclylthio und Heterocyclylamino,
wobei jeder der letztgenannten 8 Reste unsubstituiert ist oder einen oder mehrere Substituenten aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl und (C₁-C₄)Alkoxycarbonyl aufweist, substituiert ist,
oder (C₃-C₉)Cycloalkyl, Phenyl, Phenoxy, Phenylthio, Phenylcarbonyl, Heterocyclyl, Heterocyclyloxy, Heterocyclylthio oder Heterocyclylamino,
wobei jeder der letztgenannten 9 Reste unsubstituiert oder substituiert ist, vorzugsweise unsubstituiert oder durch einen oder mehrere Reste aus der Gruppe Halogen, Hydroxy, Amino, Nitro, Formyl, Carboxy, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₆)Cycloalkyl, [(C₁-C₄)Alkyl]carbonyl, [(C₁-C₄)Alkoxy]carbonyl, Aminocarbonyl, Mono(C₁-C₄)alkylamino-carbonyl und Di(C₁-C₄)alkylaminocarbonyl substituiert ist,
oder zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist.
- n: ist dabei vorzugsweise 0, 1, 2 oder 3, insbesondere 1 oder 2.
- (X)ₙ: bedeuten weiter bevorzugt n Substituenten X und dabei X jeweils unabhängig voneinander Halogen, Hydroxy, Amino, Nitro, Formyl, Carboxy, Cyano, Thiocyanato, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₆)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste, vorzugsweise bis zu drei Reste, aus der Gruppe (C₁-C₄)Alkyl, Halogen und Cyano substituiert sind, oder Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl, Phenoxy-(C₁-C₄)alkyl, Phenyl-(C₁-C₄)alkyl, Heterocyclyl, Heterocyclylamino, Heterocyclyloxy, Heterocyclylthio oder einen der letztgenannten 16 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl, (C₁-C₄)Alkoxy, substituiert ist, wobei Heterocyclyl in den Resten jeweils 3 bis 9 Ringatome und 1 bis 3 Heteroringatome aus der Gruppe N, O und S enthält, oder
zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist.
- (X)ₙ: bedeutet besonders bevorzugt n Substituenten X und dabei X jeweils unabhängig voneinander Halogen, OH, NO₂, CN, SCN (C₁-C₆)Alkyl, (C₁-C₆)Alkoxy, (C₁-C₄)Alkylcarbonyl oder (C₁-C₄)Alkyloxycarbonyl, wobei die letztgenannten vier Reste unsubstituiert oder durch Halogen oder (C₁-C₄)Alkoxy substituiert sind, und
ganz besonders bevorzugt n Substituenten X und dabei X jeweils unabhängig voneinander Halogen, Hydroxy, (C₁-C₄)Alkyl oder (C₁-C₄)Alkoxy.

Heterocyclyl bedeutet in den vorstehend oder weiter unten genannten Resten unabhängig voneinander vorzugsweise einen heterocyclischen Rest mit 3 bis 7 Ringatomen und 1 bis 3 Heteroatomen aus der Gruppe N, O und S, vorzugsweise ein heteroaromatischer Rest aus der Gruppe Pyridyl, Pyrimidinyl, Pyridazinyl, Pyrazinyl, Triazinyl, Thienyl, Thiazolyl, Thiadiazolyl, Oxazolyl, Isoxazolyl, Furyl, Pyrrolyl, Pyrazolyl, Imidazolyl und Triazolyl oder ein partiell oder vollständig hydrierter heterocylischer Rest aus der Gruppe Oxiranyl, Oxetanyl, Oxolanyl (= Tetrahydrofuryl), Oxanyl, Pyrrolidyl, Piperidyl, Piperazinyl, Dioxolanyl, Oxazolinyl, Isoxazolinyl, Oxazolidinyl, Isoxazolidinyl und Morpholinyl. Besonders bevorzugt bedeutet Heterocyclyl einen heterocyclischen Rest mit 3 bis 6 Ringatomen und einem (1) Heteroatom aus der Gruppe N, O und S, insbesondere dabei einem heteroaromatischen Rest mit 5 oder 6 Ringatomen oder einem gesättigten oder teilweise ungesättigten heterocyclischen (nicht heteroaromatischen) Rest mit 3 bis 6 Ringatomen.

Außerdem bedeutet Heterocyclyl bevorzugt einen heterocyclischen Rest mit 5 oder 6 Ringatomen und mit 2 oder 3 Heteroatomen aus der Gruppe N, O und S, insbesondere Pyrimidinyl, Pyridazinyl, Pyrazinyl, Triazinyl, Thiazolyl, Thiadiazolyl, Oxazolyl, Isoxazolyl, Pyrazolyl, Imidazolyl, Triazolyl oder Piperazinyl, Dioxolanyl, Oxazolinyl, Isoxazolinyl, Oxazolidinyl, Isoxazolidinyl oder Morpholinyl.

Insbesondere ist die Gesamtzahl der C-Atome der Reste A¹ und A²-R² zusammen eine gemäß der genannten Alternative a).

Die zusammengesetzte Gruppe -A²-R² ist vorzugsweise Cyclopropyl (hiemach auch "c-Pr"), CH₂-c-Pr, -(CH₂)₂-c-Pr, Cyclobutyl (hiemach auch "c-Bu"), CH₂-c-Bu; (CH₂)₂-c-Bu,

Gegenstand der vorliegenden Erfindung sind auch Verfahren zur Herstellung der Verbindungen der allgemeinen Formel (I) oder deren Salze, dadurch gekennzeichnet, daß man
a) eine Verbindung der Formel (II),

   R¹ - Fu (II)

   worin Fu eine funktionelle Gruppe aus der Gruppe Carbonsäureester, Carbonsäureorthoester, Carbonsäurechlorid, Carbonsäureamid, Carbonsäureanhydrid und Trichlormethyl bedeutet, mit einer Verbindung der Formel (III) oder einem Säureadditionssalz hiervon 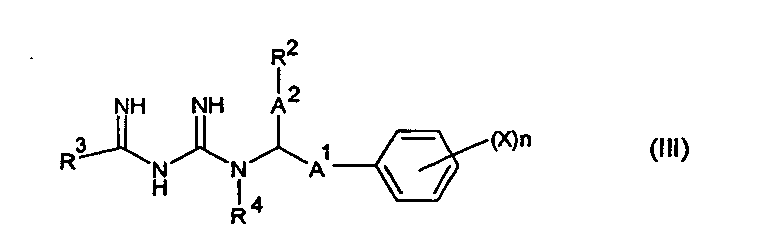 umsetzt oder
b) eine Verbindung der Formel (IV), 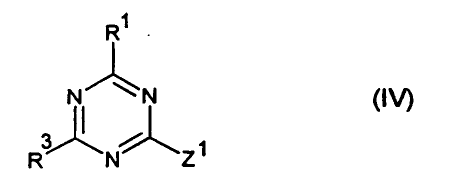
worin Z¹ einen austauschfähigen Rest oder eine Abgangsgruppe, z.B. Chlor, Trichlormethyl, (C₁-C₄)Alkylsulfonyl und unsubstituiertes oder substituiertes Phenyl-(C₁-C₄)alkylsulfonyl oder (C₁-C₄)Alkyl-phenylsulfonyl, bedeutet, mit einem geeigneten Amin der Formel (V) oder einem Säureadditionssalz hiervon 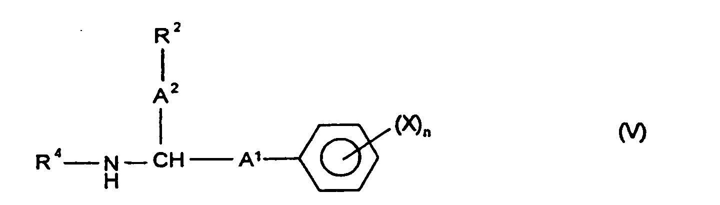 umsetzt,
wobei in den Formeln (11), (III), (IV) und (V) die Reste R¹, R², R³, R⁴, A¹, A² und X sowie n wie in Formel (I) definiert sind.

Die Umsetzung der Verbindungen der Formel (II) und (III) erfolgt vorzugsweise basenkatalysiert in einem inerten organischen Lösungsmittel, wie z.B. Tetrahydrofuran (THF), Dioxan, Acetonitril, Dimethylformamid (DMF), Methanol und Ethanol, bei Temperaturen zwischen -10 °C und dem Siedepunkt des Lösungsmittel, vorzugsweise bei 20 °C bis 60 °C; falls Säureadditionssalze der Formel (III) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicylco[5.4.0]undec-7-en (DBU). Die jeweilige Base wird dabei beispielsweise im Bereich von 0,1 bis 3 Moläquivalenten bezogen auf die Verbindung der Formel (III) eingesetzt. Die Verbindung der Formel (II) kann im Verhältnis zur Verbindung der Formel (III) beispielsweise äquimolar oder mit bis zu 2 Moläquivälenten Überschuß eingesetzt werden. Grundsätzlich sind die entsprechenden Verfahren in der Literatur bekannt (vergleiche: Comprehensive Heterocyclic Chemistry, A.R. Katritzky, C.W. Rees, Pergamon Press, Oxford, New York, 1984, Vol.3; Part 2B; ISBN 0-08-030703-5, S.290).

Die Umsetzung der Verbindungen der Formel (IV) und (V) erfolgt vorzugsweise basenkatalysiert in einem inerten organischen Lösungsmittel, wie z.B. THF, Dioxan, Acetonitril, DMF, Methanol und Ethanol, bei Temperaturen zwischen -10 °C und dem Siedepunkt des jeweiligen Lösungsmittels oder Lösungsmittelgemisches, vorzugsweise bei 20 °C bis 60 °C, wobei die Verbindung (V), falls als Säureadditionssalz eingesetzt, gegebenenfalls in situ mit einer Base freigesetzt wird. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in der Regel im Bereich von 1 bis 3 Moläquivalenten bezogen auf die Verbindung der Formel (IV) eingesetzt. Die Verbindung der Formel (IV) kann beispielsweise äquimolar zur Verbindung der Formel (V) oder mit bis zu 2 Moläquvalenten Überschuß eingesetzt werden. Grundsätzlich sind die entsprechenden Verfahren aus der Literatur bekannt (vgl. Comprehensive Heterocyclic Chemistry, A.R. Katritzky, C.W. Rees, Pergamon Press, Oxford, New York, 1984, Vol.3; Part 2B; ISBN 0-08-030703-5, S. 482).

Die Edukte der Formeln (II), (III), (IV) und (V) sind entweder kommerziell erhältlich oder können nach oder analog literaturbekannten Verfahren hergestellt werden. Einige der Verbindungen der Formel (III) und (V) sind neu und ebenfalls Gegenstand der Erfindung. Die Verbindungen können beispielsweise auch nach einem der nachfolgend beschriebenen Verfahren hergestellt werden.

Die Verbindung der Formel (IV), oder eine direkte Vorstufe davon, läßt sich beispielweise wie folgt herstellen:
1. Durch Reaktion einer Verbindung der Formel (II) mit einem Amidino-thioharnstoff-Derivat der Formel (VI), 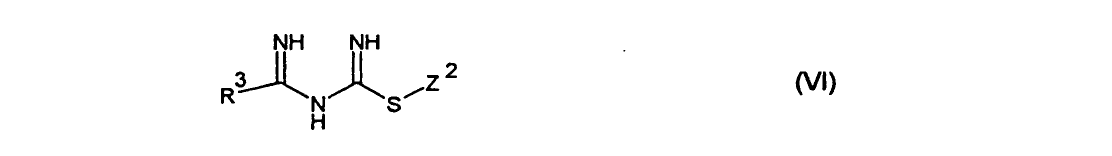 worin Z² (C₁-C₄)-Alkyl oder Phenyl-(C₁-C₄)-alkyl bedeutet und R³ wie in Formel (I) definiert sind, werden Verbindungen der Formel (IV) erhalten, in denen Z¹ = -SZ² bedeutet.
2. Durch Umsetzung eines Amidins der Formel (VII) oder eines Säureadditionssalzes davon,

   H₂N-CR¹=NH (VII)

   worin R¹ wie in Formel (I) definiert ist,
   mit einem N-Cyanodithioiminocarbonat der Formel (VIII),

   NC-N=C(S-Z³)₂ (VIII)

   worin Z³ (C₁-C₄)Alkyl oder Phenyl-(C₁-C₄)alkyl bedeutet, werden Verbindungen der Formel (IV) erhalten, worin Z¹ = -S-Z³ bedeutet.
3. Durch Umsetzung eines Alkali-dicyanamids mit einem Carbonsäurederivat der genannten Formel (II) werden Verbindungen der Formel (IV) erhalten, worin Z¹ = NH₂ bedeutet,
4. Durch Umsetzung von Trichloracetonitril mit einem Nitril der Formel (IX),

   R¹ - CN (IX)
worin R¹ wie in Formel (I) definiert ist, werden zunächst Verbindungen der Formel (X), 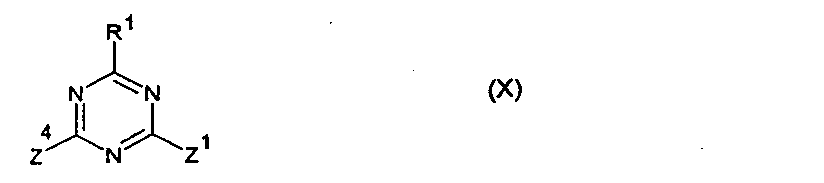 worin Z¹ und Z⁴ jeweils CCl₃ bedeuten, erhalten, welche durch nachfolgende Umsetzung mit Verbindungen der Formel H-R³ (R³ wie in Formel (I)), zu Verbindungen der Formel (IV), worin Z¹ = CCl₃ bedeutet, führen.

Die Umsetzung der Carbonsäurederivate der Formel (II) mit den Amidinothiohamstoff-Derivaten der Formel (VI) erfolgt vorzugsweise basenkatalysiert in einem organischen Lösungsmittel, wie z.B. Aceton, THF, Dioxan, Acetonitril, DMF, Methanol, Ethanol, bei Temperaturen von -10 °C bis zum Siedepunkt des Lösungsmittel, vorzugsweise bei 0 °C bis 20 °C. Die Umsetzung kann aber auch in Wasser oder in wässrigen Lösungsmitttelgemischen mit einem oder mehreren der obengenannten organischen Lösungsmitteln erfolgen. Falls (VI) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei z.B. im Bereich von 1 bis 3 Moläquivalenten bezogen auf die Verbindung der Formel (VI) eingesetzt. Verbindungen der Formel (II) und (VI) können beispielsweise äquimolar oder mit bis zu 2 Moläquivalenten Überschuß an Verbindung der Formel (II) eingesetzt werden. Grundsätzlich sind die entsprechenden Verfahren literaturbekannt (Vergl.: H. Eilingsfeld, H. Scheuermann, Chem. Ber.; 1967, 100, 1874), die entsprechenden Zwischenprodukte der Formel (IV) sind neu.

Die Umsetzung der Amidine der Formel (VII) mit den N-Cyanodithioiminocarbonaten der Formel (VIII) erfolgt vorzugsweise basenkatalysiert in einem inerten organischen Lösungsmittel, wie z.B. Acetonitril, DMF, Dimethylacetamid (DMA), N-Methylpyrrolidon (NMP), Methanol und Ethanol, bei Temperaturen von -10 °C bis zum Siedepunkt des Lösungsmittels, vorzugsweise bei 20 °C bis 80 °C. Falls (VII) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei z. B. in Bereich von 1 bis 3 Moläquivalenten bezogen auf die Verbindung der Formel (VIII) eingesetzt, Verbindungen der Formel (VII) und (VIII) können in der Regel äquimolar oder mit 2 Moläquivalenten Überschuß an Verbindung der Formel (VII) eingesetzt werden. Grundsätzlich sind die entsprechenden Verfahren literaturbekannt (vergl.: T.A. Riley, W.J. Henney, N.K. Dalley, B.E. Wilson, R.K. Robins; J. Heterocyclic Chem.; 1986, 23 (6), 1706-1714), die entsprechenden Zwischenprodukte der Formel (IV) sind neu.

Die Herstellung von Zwischenprodukten der Formel (X) mit Z¹ = Chlor kann durch Reaktion von Alkali-dicyanamid mit einem Carbonsäurederivat der Formel (II), wobei dann Fu bevorzugt die funktionelle Gruppe Carbonsäurechlorid oder Carbonsäureamid bedeutet, erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -10 °C und dem Siedepunkt des Lösungsmittels, vorzugsweise bei 20 °C bis 80 °C, wobei die entstehenden Intermediate in situ mit einem geeigneten Chlorierungsreagenz wie beispielsweise Phosphoroxychlorid chloriert werden können. Geeignete Säuren sind z.B. Halogenwasserstoffsäuren, wie HCl, oder auch Lewis-Säuren, wie z.B. AlCl₃ oder BF₃ (vergl. US-A-5095113, DuPont).

Die Herstellung von Zwischenprodukten der Formel (X) mit Z¹, Z⁴ = Trihalogenmethyl kann durch Reaktion der entsprechenden Trihalogenessigsäurenitrile mit einem Carbonsäurenitril der Formel (IX) erfolgen.
Die Umsetzung der Reaktionskomponenten erfolgt beispielsweise säurekatalysiert in einem inerten organischen Lösungsmittel wie z,B. Toluol, Chlorbenzol, chlorierten Kohlenwasserstoffen bei Temperaturen zwischen -40 °C und dem Siedepunkt des Lösungsmittels, vorzugsweise bei -10 °C bis 30 °C. Geeignete Säuren sind z.B. Halogenwasserstoffsäuren wie HCl oder auch Lewis-Säuren wie z.B. AlCl₃ oder BF₃ (vgl. EP-A-130939, Ciba Geigy).

Zwischenprodukte der Formel (IV), worin Z¹ = (C₁-C₄)Alkylmercapto oder unsubstituiertes Phenyl-(C₁-C₄)-alkylmercapto ist, können in einem inerten organischen Lösungsmittel wie z.B. Toluol, Chlorbenzol, chlorierten Kohlenwasserstoffen oder anderen bei Temperaturen zwischen -40 °C und dem Siedepunkt des Lösungsmittel, vorzugsweise bei 20 °C bis 80 °C, mit einem geeigneten Chlorierungsreagenz wie z.B. elementarem Chlor oder Phosphoroxychlorid zu reaktionsfähigeren Chlortriazinen der Formel (IV), worin Z¹ = Cl ist, überführt werden (vgl. J.K. Chakrabarti, D.E. Tupper; Tetrahedron 1975, 31(16), 1879-1882).

Zwischenprodukte der Formel (IV), wobei Z¹ = (C₁-C₄)Alkylmercapto oder unsubstituiertes oder substituiertes Phenyl-(C₁-C₄)-alkylmercapto oder (C₁-C₄)Alkylphenylthio ist, können in einem geeigneten Lösungsmittel wie z.B. chlorierten Kohlenwasserstoffen, Essigsäure, Wasser, Alkoholen, Aceton oder Mischungen hiervon bei Temperaturen zwischen 0 °C und dem Siedepunkt des Lösungsmittels, vorzugsweise von 20 °C bis 80 °C, mit einem geeigneten Oxidationssreagenz wie z.B. m-Chlorperbenzoesäure, Wasserstoffperoxid, Kaliumperoxomonosulfat oxidiert werden (vergl.: T.A. Riley, W.J. Henney, N.K. Dalley, B.E. Wilson, R.K. Robins; J. Heterocyclic Chem.; 1986, 23 (6), 1706-1714).

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphthalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzin und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.

Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat, Kalium-tert.Butylat, oder Ammoniak oder Ethanolamin. Quartäre Ammoniumsalze können z.B. durch Umsalzung oder Kondensation mit quartären Ammoniumsalzen der Formel [NRR'R"R''']⁺X⁻, worin R, R', R" und R"' unabhängig voneinander (C₁-C₄)Alkyl, Phenyl oder Benzyl bedeuten und X⁻ ein Anion, z.B. Cl⁻ bzw. OH⁻ ist, hergestellt werden.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) und deren Salze, im folgenden zusammen als (erfindungsgemäße) Verbindungen der Formel (I) bezeichnet, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.
Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.
Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen inklusive Zierpflanzungen.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Emtegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten. Vorzugsweise können die Verbindungen der Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylhamstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).

Zahlreiche molekularbiologische Techniken, mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106).

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Herbizide aus der Gruppe der Sulfonylharnstoffe, Glufosinate-ammonium oder Glyphosate-isopropylammonium und analoge Wirkstoffe resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen (I) als Herbizide zur Bekämpfung von Schadpflanzen in transgenen Kulturpflanzen.

Die erfindungsgemäße Verwendung zur Bekämpfung von Schadpflanzen oder zur Wachstumsregulierung von Pflanzen schließt auch den Fall ein, bei dem der Wirkstoff der Formel (I) oder dessen Salz erst nach der Ausbringung auf der Pflanze, in der Pflanze oder im Boden aus einer Vorläufersubstanz ("Prodrug") gebildet wird.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasserin-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z. B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether; Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt. Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0,1 bis 99 Gew.-%, insbesondere 0,1 bis 95 Gew.-%, Wirkstoff der Formel (I).
In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-% .

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 10th edition, The British Crop Protection Council and the Royal Soc. of Chemistry, 1994 und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet): acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; azimsulfurone (DPX-A8947); aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; cafenstrole (CH-900); carbetamide; cafentrazone (ICI-A0051); CDAA, d.h. 2-Chlor-N,N-di-2-propenylacetamid; CDEC, d.h. Diethyldithiocarbaminsäure-2-chlorallylester; chlomethoxyfen; chloramben; chlorazifop-butyl, chlormesulon (ICI-A0051); chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clodinafop und dessen Esterderivate (z. B. clodinafop-propargyl); clomazone; clomeprop; cloproxydim; clopyralid; cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-DB; dalapon; desmedipham; desmetryn; di-allate; dicamba; dichlobenil; dichlorprop; diclofop und dessen Ester wie diclofop-methyl; diethatyl; difenoxuron; difenzoquat; diflufenican; dimefuron; dimethachlor; dimethametryn; dimethenamid (SAN-582H); dimethazone, clomazon; dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1H-tetrazol-1-yl]-phenyl]-ethansulfonamid; ethoxyfen und dessen Ester (z.B. Ethylester, HN-252); etobenzanid (HW 52); fenoprop; fenoxan, fenoxaprop und fenoxaprop-P sowie deren Ester, z.B. fenoxaprop-P-ethyl und fenoxaprop-ethyl; fenoxydim; fenuron; flamprop-methyl; flazasulfuron; fluazifop und fluazifop-P und deren Ester, z.B. fluazifop-butyl und fluazifop-P-butyl; fluchloralin; flumetsulam; flumeturon; flumiclorac und dessen Ester (z.B. Pentylester, S-23031); flumioxazin (S-482); flumipropyn; flupoxam (KNW-739); fluorodifen; fluoroglycofen-ethyl; flupropacil (UBIC-4243); fluridone; flurochloridone; fluroxypyr; flurtamone; fomesafen; fosamine; furyloxyfen; glufosinate; glyphosate; halosafen; halosulfuron und dessen Ester (z.B. Methylester, NC-319); haloxyfop und dessen Ester; haloxyfop-P (= R-haloxyfop) und dessen Ester; hexazinone; imazamethabenz-methyl; imazapyr; imazaquin und Salze wie das Ammoniumsalz; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metabenzuron, methobenzuron; metobromuron; metolachlor; metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1-methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiargyl (RP-020630); oxadiazon; oxyfluorfen; paraquat; pebulate; pendimethalin; perfluidone; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Ester; propazine; propham; propisochlor; propyzamide; prosulfalin; prosulfocarb; prosulfuron (CGA-152005); prynachlor; pyrazolinate; pyrazon; pyrazosulfuron-ethyl; pyrazoxyfen; pyridate; pyrithiobac (KIH-2031); pyroxofop und dessen Ester (z.B. Propargylester); quinclorac; quinmerac; quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy)-propansäure und - methylester; sulfentrazon (FMC-97285, F-6285); sulfazuron; sulfometuron-methyl; sulfosate (ICI-A0224); TCA; tebutam (GCP-5544); tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiazafluron; thizopyr (Mon-13200); thidiazimin (SN-24085); thifensulfuron-methyl; thiobencarb; tiocarbazil; tralkoxydim; tri-allate; triasulfuron; triazofenamide; tribenuron-methyl; triclopyr; tridiphane; trietazine; trifluralin; triflusulfuron und Ester (z.B. Methylester, DPX-66037); trimeturon; tsitodef; vernolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

In den folgenden Beispielen beziehen sich Mengenangaben (auch Prozentangaben) auf das Gewicht, sofern nichts anderes speziell angegeben ist.

### A. Chemische Beispiele

### Beispiel A1

### 2-Amino-4-(1-fluor-1-methyl-ethyl)-6-(3-phenyl-1-cyclobutyl-1-propylamino)-1,3,5-triazin (siehe Tabelle 4, Beispiel 4-2)

Zu 1,90 g (0,00613 mol) 3-Phenyl-1-cyclobutyl-1-(biguanidino)-propan-hydrochlorid in 30 ml Methanol und 2 g Molekularsieb 3 Å (Ångström) fügt man eine aus 0,32 g (0,014 mol) Natrium und 10 ml Methanol hergestellte Lösung. Danach tropft man 1,10 g (0,0092 mol) 1-Fluor-1-methyl-propionsäuremethylester hinzu und rührt zunächst 2 Stunden bei 25°C und dann 4 Stunden bei 65°C. Die Reaktionsmischung wird filtriert, das Filtrat eingeengt und der Rückstand in Essigester aufgenommen. Es wird mit Wasser gewaschen und mit Natriumsulfat getrocknet. Das Trockenmittel wird abgesaugt und das Lösungsmittel im Vakuum eingedampft. Nach Reinigung mit Säulenchromatographie (Laufmittel: Essigsäureethylester) erhält man 1,66 g (79 % d.Th.) 2-Amino-4-(1-fluor-1-methyl-ethyl)-6-(3-phenyl-1-cyclobutyl-1-propylamino)-1,3,5-triazin.

### Beispiel A2

### 2-Amino-4-(1-fluor-1-methyl-ethyl)-6-(1-phenyl-4-cyclopropyl-4-butylamino)-1,3,5-triazin (siehe Beispiel 22-12, Tabelle 22)

1,52 g (0,008 mol) 2-Amino-4-chlor-6-(1-fluor-1-methyl-ethyl)-1,3,5-triazin und 1,64 g (0,012 mol) Kaliumcarbonat werden in 30 ml Acetonitril vorgelegt. Zu dieser Lösung tropft man 1,50 g (0,008 mol) 4-Phenyl-1-cyclopropyl-1-butylamin, gelöst in 10 ml Acetonitril, hinzu. Man läßt drei Stunden am Rückfluß kochen. Danach werden die festen Bestandteile abgesaugt und das Filtrat einrotiert. Der Rückstand wird mittels Säulenchromatographie (Laufmittel: Essigsäuremethylester) gereinigt. Man erhält 2,36 g (86 % d.Th.) 2-Amino-4-(1-fluor-1-methyl-ethyl)-6-(1-phenyl-4-cyclopropyl-4-butylamino)-1,3,5-triazin.

### Beispiel A4

### 2-Amino-6-methyl-4-[3-(3-methylphenyl)-1-cyclobutyl-1-propylamino]-1,3,5-triazin (siehe Tabelle 4, Bsp. 4-29)

2,2 g (0,015 mol) 2-Amino-4-chlor-6-methyl-1,3,5-triazin und 4,1 g (0,03 mol) K₂CO₃ werden in 50 ml Acetonitril vorgelegt. Zu dieser Lösung tropft man 2,5 g (0,015 mol) 3-(3-Methylphenyl)-1-cyclobutyl-1-propylamin, gelöst in 20 ml Acetonitril, hinzu. Anschließend läßt man 3 Stunden am Rückfluß kochen. Danach werden die festen Bestandteile abgesaugt und das Filtrat einrotiert. Der Rückstand wird mittels Säulenchromatographie (Laufmittel: Essigsäureethylester) gereinigt. Man erhält 4,3 g (92 % d.Th.) 2-Amino-6-methyl-4-[3-(3-methylphenyl)-1-cyclobutyl-1-propylamino]-1,3,5-triazin.

### Beispiel A5

### 2-Amino-4-(1-fluor-1-methyl-ethyl)-6-[4-(3,5-dimethylphenyl)-1-cyclopropyl-1-butylamino]-1,3,5-triazin (siehe Tabelle 22, Bsp. 22-28)

Zu 8,4 g (0,025 mol) 4-(3,5-Dimethylphenyl)-1-cyclopropyl-1-(1-biguanidino)-butanhydrochlorid in 50 ml Methanol und 7 g gemahlenem Molekularsieb 3Å fügt man eine aus 1,2 g (0,05 mol) Natrium und 100 ml Methanol hergestellte Methanolatlösung. Danach gibt man 5,4 g (0,045 mol) 1-Fluor-1-methylpropionsäuremethylester hinzu und rührt 2 Stunden bei 25°C und dann 4 Stunden bei 65°C. Die Reaktionsmischung wird filtriert, das Filtrat eingeengt und der Rückstand in Essigester aufgenommen. Es wird mit Wasser gewaschen und mit Natriumsulfat getrocknet. Das Trockenmittel wird abfiltriert und das Lösungsmittel im Vakuum eingedampft. Nach Reinigung mit Säulenchromatographie (Laufmittel: Essigsäureethylester) erhält man 7,7 g (83 % d.Th.) 2-Amino-4-(1-fluor-1-fluor-1-methyl-ethyl)-6-[3-(3,5-dimethyl)-1-cyclopropyl-1-butylamino]-1,3,5-triazin.

Die in den nachfolgenden Tabellen 1 bis 44 beschriebenen Verbindungen erhält man gemäß oder analog zu den vorstehenden Beispielen A1 bis A5 oder den weiter oben allgemein beschriebenen Methoden. In den Tabellen bedeuten:

### Abkürzungen:

- Me =: Methyl
- Et =: Ethyl
- Pr =: Propyl
- i-Pr =: Isopropyl
- c-Pr =: Cyclopropyl
- c-Bu =: Cyclobutyl
- t-Bu =: tertiär-Butyl
- c-Hexyl =: Cyclohexyl
- A1 =: (CH₂)₁ = -CH₂-
- A2 =: (CH₂)₂ = -CH₂CH₂-
- A3 =: (CH₂)₃ = -CH₂CH₂CH₂-
- A4 =: (CH₂)₄ = -CH₂CH₂CH₂CH₂-
- Ac =: COCH₃ = Acetyl
- Ox =: 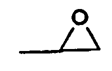 = Oxiranyl
- Ph =: Phenyl
- (X)ₙ =: "-" entspricht n = 0

Die nachfolgenden Tabellen 4, 22, 28, 32, 35, 42 und 43 beziehen sich auf die allgemeine Formel (1)

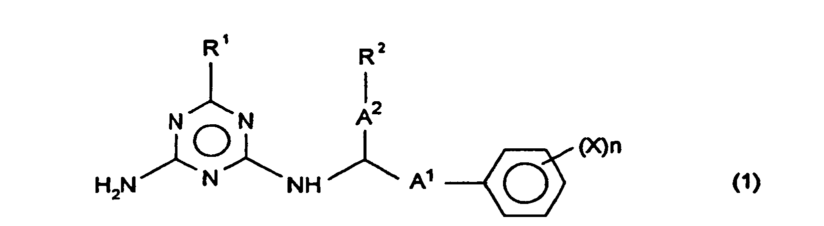

**Tabelle 4**

| Nr. | R¹ | -A²-R² | A¹ | (X)ₙ | Phys. Daten |
|---|---|---|---|---|---|
| 4-1 | i-Pr | c-Bu | A2 | - | Oel |
| 4-2 | CFMe₂ | c-Bu | A2 | - | Oel |
| 4-3 | Me | c-Bu | A2 | - | Oel |
| 4-4 | Et | c-Bu | A2 | - | |
| 4-5 | Pr | c-Bu | A2 | - | |
| 4-6 | Bu | c-Bu | A2 | - | |
| 4-7 | Ph | c-Bu | A2 | - | |
| 4-8 | CH₂-C₆H₅ | c-Bu | A2 | - | |
| 4-9 | c-Pr | c-Bu | A2 | - | |
| 4-10 | i-Pr | c-Bu | A2 | 3-Cl | Oel |
| 4-11 | CFMe₂ | c-Bu | A2 | 3-Cl | Oel |
| 4-12 | CF₃ | c-Bu | A2 | 3-Cl | |
| 4-13 | CF₃ | c-Bu | A2 | - | |
| 4-14 | i-Pr | c-Bu | A2 | 3-Me | Oel |
| 4-15 | CFMe₂ | c-Bu | A2 | 3-Me | Oel |
| 4-16 | CF₃ | c-Bu | A2 | 3-Me | |
| 4-17 | CCl₃ | c-Bu | A2 | 3-Me | |
| 4-18 | Me | c-Bu | A2 | 2-Me | |
| 4-19 | Et | c-Bu | A2 | 2-Me | |
| 4-20 | CH₂-i-Pr | c-Bu | A2 | 2-Me | |
| 4-21 | C₆H₅ | c-Bu | A2 | 2,4-Cl₂ | |
| 4-22 | CH₂-Ph | c-Bu | A2 | 4-NO₂ | |
| 4-23 | i-Pr | c-Bu | A2 | 3-OMe | Oel |
| 4-24 | CFMe₂ | c-Bu | A2 | 3-OMe | Oel |
| 4-25 | CFMe₂ | c-Bu | A2 | 2-Me | Oel |
| 4-26 | i-Pr | c-Bu | A2 | 2-Me | Oel |
| 4-27 | i-Pr | c-Bu | A2 | 3-F | Oel |
| 4-28 | CFMe₂ | c-Bu | A2 | 3-F | Oel |
| 4-29 | Me | c-Bu | A2 | 3-Me | Oel |

**Tabelle 22**

| Nr. | R¹ | -A²-R² | A¹ | (X)ₙ | Phys. Daten |
|---|---|---|---|---|---|
| 22-1 | CHClMe | c-Pr | A3 | - | |
| 22-2 | CHClMe | c-Pr | " | - | |
| 22-3 | CHFMe | c-Pr | " | - | Oel |
| 22-4 | CF₂CF₃ | c-Pr | " | - | |
| 22-5 | CF₂CHF₂ | c-Pr | " | 3-NO₂ | |
| 22-6 | CF₃ | c-Pr | " | 2,4-Cl₂ | Oel |
| 22-7 | CCl₃ | c-Pr | " | - | |
| 22-8 | Me | c-Pr | " | - | Oel |
| 22-9 | Et | c-Pr | " | - | Oel |
| 22-10 | Pr | c-Pr | " | - | |
| 22-11 | i-Pr | c-Pr | " | - | Oel |
| 22-12 | CFMe₂ | c-Pr | " | - | Oel |
| 22-13 | CH₅ | c-Pr | " | - | |
| 22-14 | CFMe₂ | c-Pr | " | 2-Cl | |
| 22-15 | i-Pr | c-Pr | " | 2-Cl | |
| 22-16 | CFMe₂ | c-Pr | " | 2,4-Cl₂ | |
| 22-17 | i-Pr | c-Pr | " | 2,4-Cl₂ | |
| 22-18 | CFMe₂ | c-Pr | " | 3-Cl | Oel |
| 22-19 | i-Pr | c-Pr | " | 3-Cl | |
| 22-20 | i-Pr | c-Pr | " | 3,5-Cl₂ | |
| 22-21 | CFMe₂ | c-Pr | " | 3,5-Cl₂ | |
| 22-23 | CFMe₂ | c-Pr | " | 2-F | |
| 22-24 | CFMe₂ | c-Pr | " | 3-F | Oel |
| 22-25 | i-Pr | c-Pr | " | 3-F | Oel |
| 22-26 | i-Pr | c-Pr | " | 3-Me | Oel |
| 22-27 | CFMe₂ | c-Pr | " | 3-Me | Oel |
| 22-28 | CFMe₂ | c-Pr | " | 3,5-Me₂ | Oel |
| 22-29 | i-Pr | c-Pr | " | 3-OMe | Oel |
| 22-30 | CFMe₂ | c-Pr | " | 3-OMe | Oel |
| 22-31 | CF₃ | c-Pr | " | - | Oel |

**Tabelle 28**

| Nr. | R¹ | -A²-R² | A¹ | (X)ₙ | Phys. Daten |
|---|---|---|---|---|---|
| 28-1 | Me | c-Bu | A3 | - | |
| 28-2 | Et | c-Bu | " | - | |
| 28-3 | Pr | c-Bu | " | - | |
| 28-4 | i-Pr | c-Bu | " | - | Oel |
| 28-5 | i-Bu | c-Bu | " | - | Oel |
| 28-6 | CH₂-i-Pr | c-Bu | " | - | |
| 28-7 | CF₃ | c-Bu | " | - | |
| 28-8 | CH₂F | c-Bu | " | - | |
| 28-9 | CF₂CHF₂ | c-Bu | " | - | |
| 28-10 | CFMe₂ | c-Bu | " | - | Oel |
| 28-11 | i-Pr | c-Bu | " | 4-NO₂ | |
| 28-12 | CFMe₂ | c-Bu | " | 2-CF₃ | |
| 28-13 | i-Pr | c-Bu | " | 3-Cl | Oel |
| 28-14 | CFMe₂ | c-Bu | " | 3-Cl | Oel |
| 28-15 | i-Pr | c-Bu | " | 3-CF₃ | |
| 28-16 | CFMe₂ | c-Bu | " | 3-CF₃ | |
| 28-17 | i-Pr | c-Bu | " | 3-Me | Oel |
| 28-18 | CFMe₂ | c-Bu | " | 3-Me | Oel |
| 28-18 | i-Pr | c-Bu | " | 3-F | |
| 28-19 | CFMe₂ | c-Bu | " | 3-F | |
| 28-20 | i-Pr | c-Bu | " | 3-OMe | Oel |
| 28-21 | CFMe₂ | c-Bu | " | 3-OMe | Oel |

**Tabelle 32**

| Nr. | R¹ | -A²-R² | A¹ | (X)ₙ | Phys. Daten |
|---|---|---|---|---|---|
| 32-1 | i-Pr | CH₂-c-Pr | A2 | - | Oel |
| 32-2 | CFMe₂ | CH₂-c-Pr | " | - | Oel |
| 32-3 | i-Pr | CH₂-c-Pr | " | 3-Br | |
| 32-4 | CFMe₂ | CH₂-c-Pr | " | 3-Br | |
| 32-5 | i-Pr | CH₂-c-Pr | " | 3-Cl | Oel |
| 32-6 | CFMe₂ | CH₂-c-Pr | " | 3-Cl | Oel |
| 32-7 | i-Pr | CH₂-c-Pr | " | 3-F | |
| 32-8 | CFMe₂ | CH₂-c-Pr | " | 3-F | |
| 32-9 | i-Pr | CH₂-c-Pr | " | 3-Me | Oel |
| 32-10 | CFMe₂ | CH₂-c-Pr | " | 3-Me | Oel |
| 32-11 | i-Pr | CH₂-c-Pr | " | 3-OMe | Oel |
| 32-12 | CFMe₂ | CH₂-c-Pr | " | 3-OMe | Oel |

**Tabelle 35**

| Nr. | R¹ | -A²-R² | A¹ | (X)ₙ | Phys. Daten |
|---|---|---|---|---|---|
| 35-1 | CFMe₂ | CH₂-c-Bu | A2 | - | |
| 35-2 | i-Pr | CH₂-c-Bu | " | - | |
| 35-3 | CH₃ | CH₂-c-Bu | " | - | |
| 35-4 | CF₃ | CH₂-c-Bu | " | - | |
| 35-5 | CClMe₂ | CH₂-c-Bu | " | - | |
| 35-6 | CHFMe | CH₂-c-Bu | " | - | |

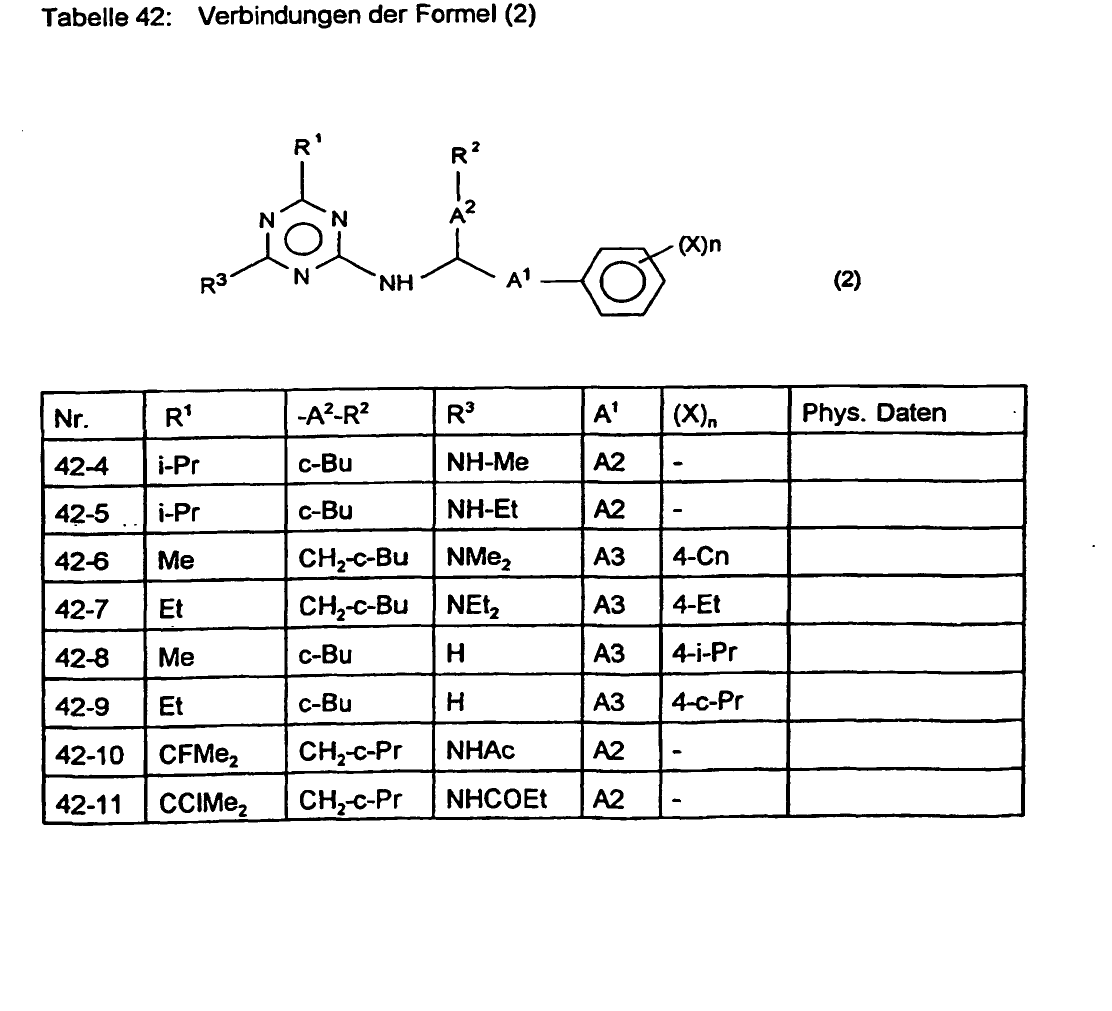

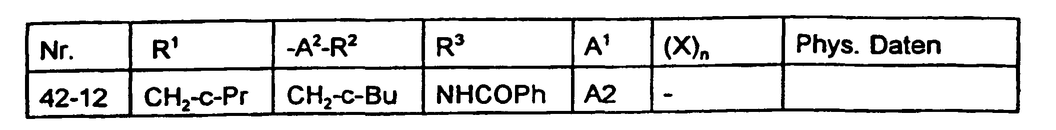

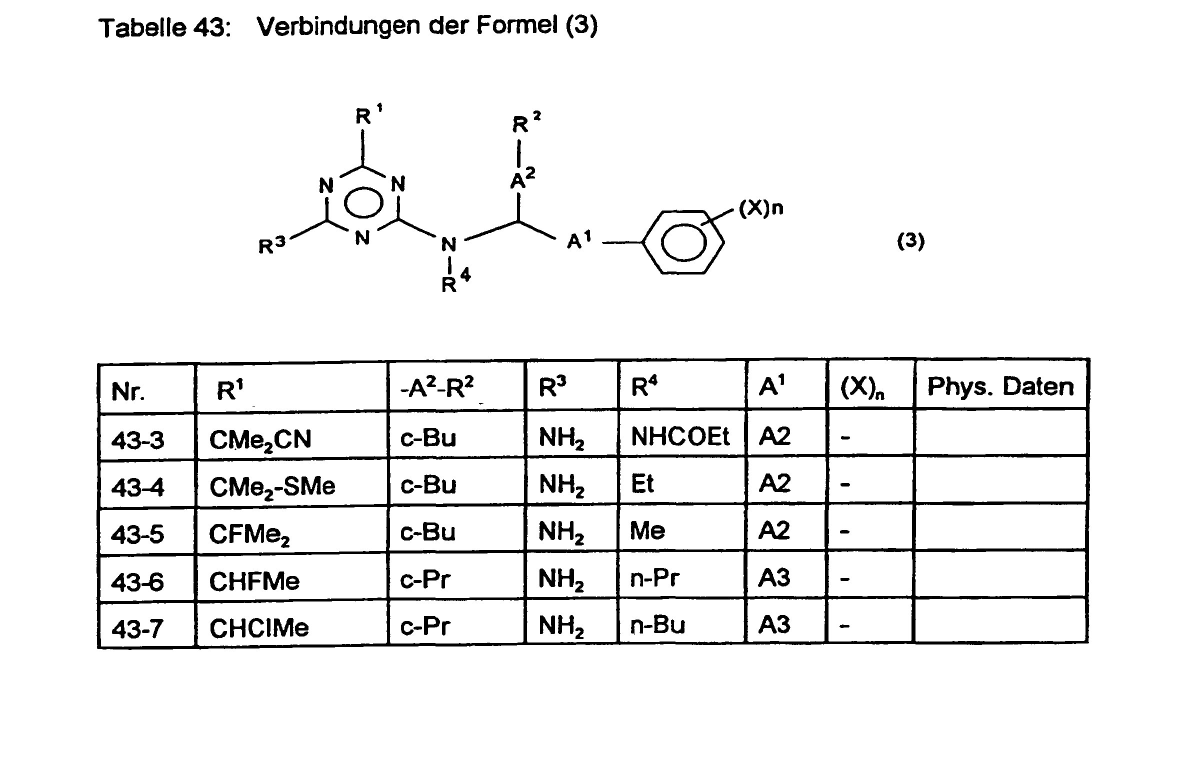

NMR-Daten zu einzelnen Beispielen:

zu Bsp. 4-2:
¹H-NMR (DMSO-d₆): δ = 1,5 (s, 3H), 1,6 (s, 3H), 1,5 - 2,0 (m), 2,4 - 2,6 (m), 4,0 (m, 1 H), 7,2 (m, 5H)
zu Bsp. 4-28:
¹H-NMR (CDCl₃): 1,6 (s, 3H), 1,7 (s, 3H), 1,5 - 1,9 (m), 2,4 (m, 2H), 2,6 - 2,7 (m, 2H), 4,1 (m, 1 H), 4,1 (m, 1 H), 6,8 - 7,0 (m, 3H), 7,2 (m, 1 H)
zu Bsp. 22-3: ¹H-NMR (DMSO-d₆): δ = 0,1 (m, 1H), 0,3 (m, 2H), 0,4 (m, 1H), 0,9 (m, 1H), 1,5 (s, 3H), 1,6 (s, 3H), 3,5 m (1H), 7,1 - 7,3 (m, 5H)
zu Bsp. 22-25: ¹H-NMR (CDCl₃): δ = 0,2 - 0,6 (m, 4H), 0,8 (m, 1H), 1,2 (d, 6H), 1,6 - 1,8 (m, 4H), 2,5 - 2,7 (m, 2H), 3,5 m (1H), 6,9 (m, 3H), 7,2 m (1 H)
zu Bsp. 28-10:
¹H-NMR (DMSO-d₆): δ = 1,5 (s, 3H), 1,6 (s, 3H), 1,5 - 1,9 (m), 2,6 (m), 4,0 (m, 1H), (m, 1 H), 7,1 - 7,3 m (5H)
zu Bsp. 32-12:
¹H-NMR (DMSO-d₆): δ = 0,1 (m, 2H), 0,4 (m, 2H), 0,7 m (1H), 1,2 (d, 6H), 1,4 (m, 3H), 1,8 (m, 2H), 2,5 - 2,7 m (2H), 3,7 (m, 3H), 4,0 (m, 1H), 6,7 (m, 3H), 7,2 m (1H)

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   - 75 Gewichtsteile: einer Verbindung der Formel (I),
   - 10 ": ligninsulfonsaures Calcium,
   - 5 ": Natriumlaurylsulfat,
   - 3 ": Polyvinylalkohol und
   - 7 ": Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   - 25 Gewichtsteile: einer Verbindung der Formel (I),
   - 5 ": 2,2'-dinaphthylmethan-6,6'-disulfonsaures Natrium
   - 2 ": oleoylmethyltaurinsaures Natrium,
   - 1 Gewichtsteil: Polyvinylalkohol,
   - 17 Gewichtsteile: Calciumcarbonat und
   - 50 ": Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise zeigen die Beispiele Nr. 4-1, 4-2, 4-3, 4-10, 4-11, 4-14, 4-15, 4-23, 4-24, 4-25, 4-26, 4-27, 4-28, 4-29, 22-3, 22-6, 22-8, 22-9, 22-11, 22-12, 22-18, 22-24, 22-25, 22-26, 22-27, 22-28, 22-29, 22-30, 28-4, 28-5, 28-10, 28-13, 28-14, 28-17, 28-18, 28-20, 28-21, 32-1, 32-2, 32-5, 32-6, 32-9, 32-10, 32-11 und 32-12, im Test sehr gute herbizide Wirkung gegen Schadpflanzen wie Stellaria media, Lolium multiflorum, Amaranthus retroflexus, Sinapis alba, Avena sativa und Setaria viridis im Vorauflaufverfahren bei einer Aufwandmenge von 1 kg oder weniger Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen werden in verschiedenen Dosierungen mit einer Wasseraufwandmenge von umgerechnet 600 bis 800 l/ha auf die grünen Pflanzenteile gesprüht. Nach ca. 3 bis 4 Wochen Standzeit der Versuchspflanzen im Gewächshaus unter optimalen Wachstumsbedingungen wird die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise zeigen die Beispiele Nr. 4-1, 4-2, 4-3, 4-10, 4-11, 4-14, 4-15, 4-23, 4-24, 4-25, 4-26, 4-27, 4-28, 4-29, 22-3, 22-6, 22-8, 22-9, 22-11, 22-12, 22-18, 22-24, 22-25, 22-26, 22-27, 22-28, 22-29, 22-30, 28-4, 28-5, 28-10, 28-13, 28-14, 28-17, 28-18 28-20, 28-21, 32-1, 32-2, 32-5, 32-6, 32-9, 32-10, 32-11 und 32-12, (s. Tabellen) im Test sehr gute herbizide Wirkung gegen Schadpflanzen wie Sinapis alba, Echinochloa crus-galli, Lolium multiflorum, Stellaria media, Cyperus iria, Amaranthus retroflexus, Setaria viridis und Avena sativa im Nachauflaufverfahren bei einer Aufwandmenge von 1 kg und weniger Aktivsubstanz pro Hektar.

### 3. Wirkung auf Schadpflanzen in Reis

Verpflanzter und gesäter Reis sowie typische Reisunkräuter und -ungräser werden im Gewächshaus bis zum Dreiblattstadium (Echinochloa crus- galli 1,5-Blatt) unter Paddyreis-Bedingungen (Anstauhöhe des Wassers: 2 - 3 cm) in geschlossenen Plastiktöpfen angezogen. Danach erfolgt die Behandlung mit den erfindungsgemäßen Verbindungen. Hierzu werden die formulierten Wirkstoffe in Wasser suspendiert, gelöst bzw. emulgiert und mittels Gießapplikation in das Anstauwasser der Test-pflanzen in unterschiedlichen Dosierungen ausgebracht. Nach der so durchgeführten Behandlung werden die Versuchspflanzen im Gewächshaus unter optimalen Wachstumsbedingungen aufgestellt und während der gesamten Versuchszeit so gehalten.

Etwa drei Wochen nach der Applikation erfolgt die Auswertung mittels optischer Bonitur der Pflanzenschäden im Vergleich zu unbehandelten Kontrollen. Die erfindungsgemäßen Verbindungen weisen zeigen sehr gute herbizide Wirkung gegen Schadpflanzen auf. Beispielsweise zeigen die Verbindungen der Beispiele Nr. 4-1, 4-2, 4-14, 4-15, 4-23 und 4-24 (s. Tabellen) im Test sehr gute herbizide Wirkung gegen Schadpflanzen, die typisch für Reiskulturen sind, wie z.B. Cyperus monti, Echinochloa crus-galli und Sagittaria pygmaea.

### 4. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt lassen. Einige Substanzen schonen darüber hinaus auch Gramineen-Kulturen wie z.B. Gerste, Weizen, Roggen, Sorghum, Mais oder Reis. Die Verbindungen der Formel (I) zeigen teilweise eine hohe Selektivität und eignen sich deshalb zur Bekämpfung von unerwünschten Pflanzenwuchs in Iandwirtschaftlichen Kulturen.

## Patentansprüche

1. Verbindungen der Formel (I) oder deren Salze, 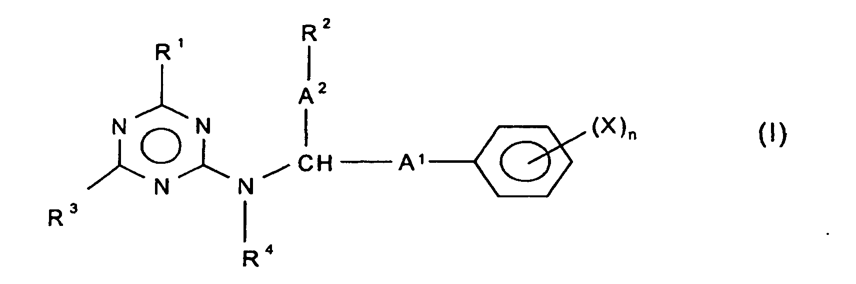 worin
R¹ Phenyl, das unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist,
oder (C₃-C₉)Cycloalkyl, das unsubstituiert oder durch einen oder mehrere Reste aus der Gruppe Halogen, Hydroxy, Amino, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist,
oder Heterocyclyl, das unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist,
oder (C₁-C₄)Alkyl, (C₂-C₆)Alkenyl oder (C₂-C₆)Alkinyl, wobei jeder der letztgenannten 3 Reste unsubstituiert oder durch einen oder mehrere Reste aus der Gruppe Halogen, Hydroxy, Cyano, Nitro, Thiocyanato, (C₁-C₄)Alkoxy, (C₁-C₄)Haloalkoxy, (C₂-C₄)Alkenyloxy, (C₂-C₄)Haloalkenyloxy, (C₁-C₄)Alkylthio, (C₁-C₄)Alkylsulfinyl, (C₁-C₄)Alkylsulfonyl, (C₁-C₄)Haloalkylsulfinyl, (C₁-C₄)Haloalkylsulfonyl und (C₃-C₆)Cycloalkyl, das unsubstituiert oder oder durch einen oder mehrere Reste aus der Gruppe Halogen, Hydroxy, Amino, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist, und Phenyl und Heterocyclyl, wobei jeder der zwei letztgenannten Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist, und Reste der Formeln R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- und R'R"NC(=Z')-NR"'-,
worin R', R" und R"' jeweils unabhängig voneinander (C₁-C₄)Alkyl, Phenyl, Phenyl-(C₁-C₄)alkyl, (C₃-C₆)Cycloalkyl oder (C₃-C₆)Cycloalkyl(C₁-C₄)alkyl, wobei jeder der 5 letztgenannten Reste unsubstituiert oder durch einen oder mehrere Reste aus der Gruppe Halogen, Hydroxy, Amino, Nitro, Formyl, Cyano Thiocyanato, (C₁-C₄)Alkoxy, (C₁-C₄)Alkylthio, Mono(C₁-C₄)alkylamino, Di(C₁-C₄)alkylamino, (C₂-C₄)Alkenyl, (C₂-C₄)Alkinyl, (C₃-C₆)Cycloalkyl und im Fall cyclischer Reste auch (C₁-C₄)Alkyl und (C₁-C₄)Haloalkyl substituiert ist, bedeuten und worin Z und Z' unabhängig voneinander jeweils ein Sauerstoff- oder Schwefelatom sind, substituiert ist,
A¹ geradkettiges Alkylen der Formel CH₂CH₂ und
A²-R² Cyclopropyl-methyl, 2-Cyclopropyl-ethyl, Cyclobutyl, Cyclobutyl-methyl oder 2-Cyclobutyl-ethyl, oder
A¹ geradkettiges Alkylen der Formel CH₂CH₂CH₂ und
A²-R² Cyclopropyl, Cyclopropyl-methyl, 2-Cyclopropyl-ethyl, Cyclobutyl, Cyclobutylmethyl oder 2-Cyclobutyl-ethyl, und
R³ Wasserstoff, (C₁-C₄)Alkyl, das unsubstituiert oder durch einen oder mehrere Reste aus der Gruppe Halogen, Hydroxy, Amino, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino und Di(C₁-C₄)alkylamino substituiert ist, oder Phenyl oder (C₃-C₆)Cycloalkyl, wobei jeder der letztgenannten 2 Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl und (C₁-C₄)Haloalkylsulfonyl substituiert ist, oder einen Rest der Formel N(B¹-D¹)(B²-D²),
R⁴ einen Rest der Formel -B³-D³, wobei B³ und D³ wie unten definiert sind,
B¹, B² und B³ unabhängig voneinander eine direkte Bindung oder eine divalente Gruppe der Formeln -C(=Z*)-, -C(=Z*)-Z**-, -C(=Z*)-NH- oder -C(=Z*)-NR*-, wobei Z* = O oder S, Z** = O oder S und R* = (C₁-C₄)Alkyl, Phenyl, Phenyl-(C₁-C₄)alkyl, (C₃-C₆)Cycloalkyl oder (C₃-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl, (C₁-C₄)Haloalkylsulfonyl und im Fall cyclischer Reste auch (C₁-C₄)Alkyl und (C₁-C₄)Haloalkyl substituiert ist;
D¹, D² und D³ unabhängig voneinander Wasserstoff, (C₁-C₆)Alkyl, Phenyl, Phenyl-(C₁-C₄)alkyl, (C₃-C₆)Cycloalkyl oder (C₃-C₆)Cycloalkyl-(C₁-C₆)alkyl, wobei jeder der 5 letztgenannten Reste unsubstituiert oder durch einen oder mehrere Reste aus der Gruppe Halogen, Hydroxy, Amino, Nitro, Formyl, Carboxy, Sulfo, Cyano, Thiocyanato, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, (C₁-C₄)Alkylsulfonyl, (C₁-C₄)Haloalkylsulfonyl und im Fall cyclischer Reste auch (C₁-C₄)Alkyl und (C₁-C₄)Haloalkyl substituiert ist,
(X)ₙ n Substituenten X und dabei X unabhängig voneinander Halogen, Hydroxy, Amino, Nitro, Formyl, Carboxy, Cyano, Thiocyanato, Aminocarbonyl oder (C₁-C₄)Alkyl, (C₁-C₄)Alkoxy, (C₁-C₄)Alkylthio, Mono(C₁-C₄)alkylamino, Di(C₁-C₄)alkylamino, (C₂-C₄)Alkenyl, (C₂-C₄)Alkinyl, [(C₁-C₄)Alkyl]carbonyl, [(C₁-C₄)Alkoxy]carbonyl, Mono(C₁-C₄)alkylamino-carbonyl, Di(C₁-C₄)alkylaminocarbonyl, N-(C₁-C₆)Alkanoyl-amino oder N-(C₁-C₄)Alkanoyl-N-(C₁-C₄)alkylamino,
wobei jeder der letztgenannten 13 Reste unsubstituiert oder durch einen oder mehrere Reste aus der Gruppe Halogen, Hydroxy, Amino, Cyano, Thiocyanato, (C₁-C₄)Alkoxy, (C₁-C₄)Haloalkoxy, (C₁-C₄)Alkylthio, Mono(C₁-C₄)alkylamino, Di(C₁-C₄)alkylamino, (C₃-C₆)Cycloalkyl, (C₃-C₆)Cycloalkylamino, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylaminocarbonyl, Phenyl, Phenoxy, Phenylthio, Phenylcarbonyl, Heterocyclyl, Heterocyclyloxy, Heterocyclylthio und Heterocyclylamino, wobei jeder der letztgenannten 8 Reste unsubstituiert ist oder einen oder mehrere Substituenten aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl und (C₁-C₄)Alkoxycarbonyl aufweist, substituiert ist, oder (C₃-C₉)Cycloalkyl, Phenyl, Phenoxy, Phenylthio, Phenylcarbonyl, Heterocyclyl, Heterocyclyloxy, Heterocyclylthio oder Heterocyclylamino, wobei jeder der letztgenannten 9 Reste unsubstituiert oder durch einen oder mehrere Reste aus der Gruppe Halogen, Hydroxy, Amino, Nitro, Formyl, Carboxy, Cyano, Thiocyanato, (C₁-C₄)Alkyl, (C₁-C₄)Haloalkyl, (C₁-C₄)Alkoxy, (C₁-C₄)Haloalkoxy, (C₁-C₄)Alkylthio, (C₁-C₄)Haloalkylthio, Mono(C₁-C₄)alkylamino, Di(C₁-C₄)alkylamino, (C₃-C₆)Cycloalkyl, [(C₁-C₄)Alkyl]carbonyl, [(C₁-C₄)Alkoxy]carbonyl, Aminocarbonyl, Mono(C₁-C₄)alkylamino-carbonyl und Di(C₁-C₄)alkylamino-carbonyl substituiert ist,
oder zwei benachbarte Reste X gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist, und
n 0, 1, 2 oder 3
bedeuten und
Heterocyclyl in den vorstehend genannten Resten unabhängig voneinander jeweils einen heterocyclischen Rest mit 3 bis 7 Ringatomen und 1 bis 3 Heteroatomen aus der Gruppe N, O und S bedeutet.

2. Verbindung der Formel (I) und deren Salze nach Anspruch 1, **dadurch gekennzeichnet, daß**
R¹ (C₁-C₄)Alkyl, das unsubstituiert oder durch einen oder mehrere Reste aus der Gruppe Halogen, (C₁-C₄)Alkoxy, (C₁-C₄)Alkylthio, (C₁-C₄)Alkylsulfonyl, (C₃-C₆)Cycloalkyl, das unsubstituiert ist, und Phenyl, das unsubstituiert oder durch einen oder mehrere Reste aus der Gruppe Halogen, (C₁-C₄)Alkyl und (C₁-C₄)Haloalkyl, (C₁-C₄)Alkoxy, (C₁-C₄)Haloalkoxy, (C₁-C₄)Alkylthio, Amino, Mono- und Di[(C₁-C₄)alkyl]amino, (C₁-C₄)Alkanoylamino, Benzoylamino, Nitro, Cyano, [(C₁-C₄)Alkyl]carbonyl, Formyl, Carbamoyl, Mono- und Di-[(C₁-C₄)alkyl]aminocarbonyl und (C₁-C₄)Alkylsulfonyl substituiert ist, und Heterocyclyl mit 3 bis 6 Ringatomen und 1 bis 3 Heteroringatomen aus der Gruppe N, O und S, wobei der Ring unsubstituiert oder durch einen oder mehrere Reste aus der Gruppe Halogen, (C₁-C₄)Alkyl und Oxo substituiert ist, substituiert ist und inklusive Substituenten 2 bis 30 C-Atome aufweist,
bedeutet.

3. Verbindung der Formel (I) und deren Salze nach Anspruch 1 oder 2, **dadurch gekennzeichnet, daß**
A¹ geradkettiges Alkylen der Formel CH₂CH₂ und
A²-R² Cyclopropyl-methyl, Cyclobutyl oder Cyclobutyl-methyl bedeuten.

4. Verbindung der Formel (I) und deren Salze nach Anspruch 1 oder 2, **dadurch gekennzeichnet, daß**
A¹ geradkettiges Alkylen der Formel CH₂CH₂CH₂ und
A²-R² Cyclopropyl, Cyclopropyl-methyl oder Cyclobutyl, Cyclobutyl-methyl
bedeuten.

5. Verbindung der Formel (I) und deren Salze nach einem der Ansprüche 1 bis 4,
**dadurch gekennzeichnet, daß**
B¹, B², und B³ unabhängig voneinander eine direkte Bindung oder eine divalente
Gruppe der Formeln -C(=Z*)-, -C(=Z*)-Z**-, -C(=Z*)-NH- oder -C(=Z*)-NR*-, wobei Z* = O oder S, Z** = O oder S und R* = (C₁-C₄)Alkyl, Phenyl, Phenyl-(C₁-C₄)alkyl, (C₃-C₆)Cycloalkyl oder (C₃-C₆)Cycloalkyl-(C₁-C₄)alkyl, wobei jeder der 5 letztgenannten Reste unsubstituiert oder durch einen oder mehrere Reste aus der Gruppe Halogen, Hydroxy, Amino, Formyl, (C₁-C₄)Alkoxy, (C₁-C₄)Haloalkoxy, (C₁-C₄)Alkylthio, Mono(C₁-C₄)alkylamino, Di(C₁-C₄)alkylamino, (C₃-C₉)Cycloalkyl, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl und im Fall cyclischer Reste auch (C₁-C₄)Alkyl und (C₁-C₄)Haloalkyl substituiert ist, und
D¹, D² und D³ unabhängig voneinander
(C₁-C₄)Alkyl, (C₃-C₆)Cycloalkyl, Phenyl oder Phenyl-(C₁-C₄)alkyl, wobei jeder der beiden letztgenannten Reste im Phenylteil unsubstituiert oder durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist,
bedeuten.

6. Verbindung der Formel (I) und deren Salze nach einem der Ansprüche 1 bis 5, **dadurch gekennzeichnet, daß**
(X)ₙ n Substituenten X und dabei X jeweils unabhängig voneinander Halogen, OH, NO₂, CN, SCN (C₁-C₆)Alkyl, (C₁-C₆)Alkoxy, (C₁-C₄)Alkylcarbonyl oder (C₁-C₄)Alkyloxycarbonyl, wobei die letztgenannten vier Reste unsubstituiert oder durch Halogen oder (C₁-C₄)Alkoxy substituiert sind,
bedeutet.

7. Verfahren zur Herstellung der Verbindungen der allgemeinen Formel (I) oder deren Salze nach einem der Ansprüche 1 bis 6, **dadurch gekennzeichnet, daß** man
a) eine Verbindung der Formel (II),
R¹ - Fu (II)
worin Fu eine funktionelle Gruppe aus der Gruppe Carbonsäureester, Carbonsäureorthoester, Carbonsäurechlorid, Carbonsäureamid, Carbonsäureanhydrid und Trichlormethyl bedeutet, mit einem Biguanidid der Formel (III) oder einem Säureadditionssalz hiervon 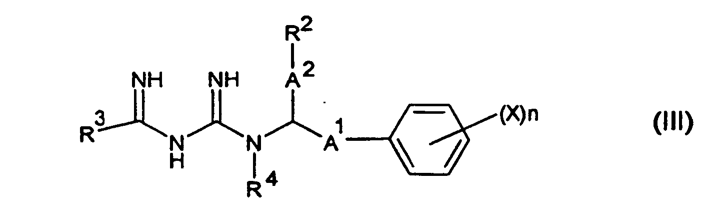 umsetzt oder
b) eine Verbindung der Formel (IV), 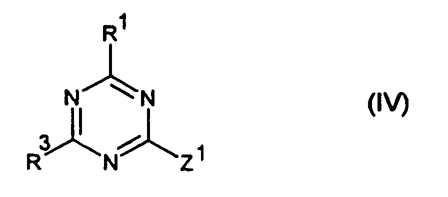
worin Z¹ einen austauschfähigen Rest oder eine Abgangsgruppe darstellt, mit einem geeigneten Amin der Formel (V) oder einem Säureadditionssalz hiervon 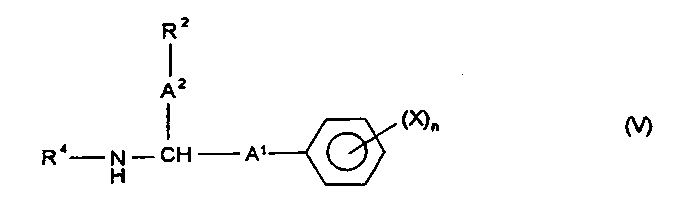 umsetzt,
wobei in den Formeln (II), (III), (IV) und (V) die Reste R¹, R², R³, R⁴, A¹, A² und X sowie n wie in Formel (I) definiert sind.

8. Herbizides oder pflanzenwachstumsregulierendes Mittel, **dadurch gekennzeichnet, daß** es eine oder mehrere Verbindungen der Formel (I) oder deren Salze nach einem der Ansprüche 1 bis 6 und im Pflanzenschutz übliche Formulierungshilfsmittel enthält.

9. Verfahren zur Bekämpfung von Schadpflanzen oder zur Wachstumsregulierung von Pflanzen, **dadurch gekennzeichnet, daß** man eine wirksame Menge von einer oder mehreren Verbindungen der Formel (I) oder deren Salzen nach einem der Ansprüche 1 bis 6 auf die Pflanzen, Pflanzensamen oder die Anbaufläche appliziert.

10. Verwendung von Verbindungen der Formel (I) oder deren Salzen nach einem der Ansprüche 1 bis 6 als Herbizide oder Pflanzenwachstumsregulatoren.

11. Verwendung nach Anspruch 10, **dadurch gekennzeichnet, daß** die Verbindungen der Formel (I) oder deren Salze zur Bekämpfung von Schadpflanzen oder zur Wachstumsregulierung in Kulturen von Nutz- oder Zierpflanzen eingesetzt werden.

12. Verwendung nach Anspruch 11, **dadurch gekennzeichnet, daß** die Kulturpflanzen transgene Kulturpflanzen sind.

13. Verbindungen der Formel (III) oder (V), wie sie nach Anspruch 7 definiert sind.

## Claims

1. A compound of the formula (I) or a salt thereof 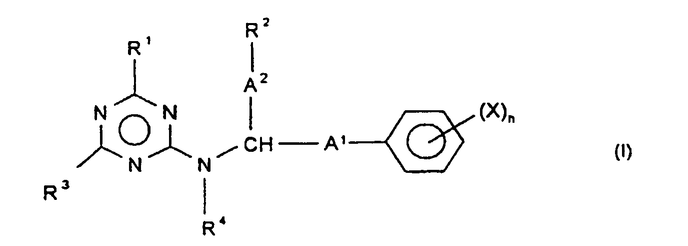 in which
R¹ is phenyl which is unsubstituted or substituted by one or more radicals selected from the group consisting of halogen, hydroxyl, amino, nitro, formyl, carboxyl, sulfo, cyano, thiocyanato, (C₁-C₄)alkyl, (C₁-C₄)haloalkyl, (C₁-C₄)alkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₉)cycloalkyl, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, (C₁-C₄)alkylsulfonyl and (C₁-C₄)haloalkylsulfonyl,
or
(C₃-C₉)cycloalkyl which is unsubstituted or substituted by one or more radicals selected from the group consisting of halogen, hydroxyl, amino, cyano, thiocyanato, (C₁-C₄)alkyl, (C₁-C₄)haloalkyl, (C₁-C₄)alkoxy, (C₁-C₄)haloalkoxy, (C₁-C₄)alkylthio, (C₁-C₄)haloalkylthio, mono(C₁-C₄)alkylamino and di(C₁-C₄)alkylamino,
or
heterocyclyl which is unsubstituted or substituted by one or more radicals selected from the group consisting of halogen, hydroxyl, amino, nitro, formyl, carboxyl, sulfonyl, cyano, thiocyanato, (C₁-C₄)alkyl, (C₁-C₄)haloalkyl, (C₁-C₄)alkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₉)cycloalkyl, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, (C₁-C₄)alkylsulfonyl and (C₁-C₄)haloalkylsulfonyl,
or
(C₁-C₄)alkyl, (C₂-C₆)alkenyl or (C₂-C₆)alkynyl, each of the last-mentioned 3 radicals being unsubstituted or substituted by one or more radicals selected from the group consisting of halogen, hydroxyl, cyano, nitro, thiocyanato, (C₁-C₄)alkoxy, (C₁-C₄)haloalkoxy, (C₂-C₄)alkenyloxy, (C₂-C₄)haloalkenyloxy, (C₁-C₄)alkylthio, (C₁-C₄)alkylsulfinyl, (C₁-C₄)alkylsulfonyl, (C₁-C₄)haloalkylsulfinyl, (C₁-C₄)haloalkylsulfonyl and (C₃-C₆)cycloalkyl which is unsubstituted or substituted by one or more radicals selected from the group consisting of halogen, hydroxyl, amino, cyano, thiocyanato, (C₁-C₄)alkyl, (C₁-C₄)haloalkyl, (C₁-C₄)alkoxy, (C₁-C₄)haloalkoxy, (C₁-C₄)alkylthio, (C₁-C₄)haloalkylthio, mono(C₁-C₄)alkylamino and di(C₁-C₄)alkylamino, and phenyl and heterocyclyl, each of the two last-mentioned radicals being unsubstituted or substituted by one or more radicals selected from the group consisting of halogen, hydroxyl, amino, nitro, formyl, carboxyl, sulfonyl, cyano, thiocyanato, (C₁-C₄)alkyl, (C₁-C₄)haloalkyl, (C₁-C₄)alkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₉)cycloalkyl, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, (C₁-C₄)alkylsulfonyl and (C₁-C₄)haloalkylsulfonyl, and radicals of the formulae R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- and R'R"N-C(=Z')-NR'''-
in which R', R" and R"' in each case independently of one another are (C₁-C₄)alkyl, phenyl, phenyl-(C₁-C₄)alkyl, (C₃-C₆)cycloalkyl or (C₃-C₆)cycloalkyl-(C₁-C₄)alkyl, each of the 5 last-mentioned radicals being unsubstituted or substituted by one or more radicals selected from the group consisting of halogen, hydroxyl, amino, nitro, formyl, cyano, thiocyanato, (C₁-C₄)alkoxy, (C₁-C₄)alkylthio, mono(C₁-C₄)alkylamino, di(C₁-C₄)alkylamino, (C₂-C₄)alkenyl, (C₂-C₄)alkynyl, (C₃-C₆)cycloalkyl and in the case of cyclic radicals also (C₁-C₄)alkyl and (C₁-C₄)haloalkyl, and in which Z and Z' independently of one another are in each case an oxygen or sulfur atom,
A¹ is straight-chain alkylene of the formula CH₂CH₂ and
A²-R² is cyclopropylmethyl, 2-cyclopropylethyl, cyclobutyl, cyclobutylmethyl or 2-cyclobutylethyl, or
A¹ is straight-chain alkylene of the formula CH₂CH₂CH₂ and
A²-R² is cyclopropyl, cyclopropylmethyl, 2-cyclopropylethyl, cyclobutyl, cyclobutylmethyl or 2-cyclobutylethyl, and
R³ is hydrogen, (C₁-C₄)alkyl which is unsubstituted or substituted by one or more radicals selected from the group consisting of halogen, hydroxyl, amino, cyano, thiocyanato, (C₁-C₄)alkoxy, (C₁-C₄)haloalkoxy, (C₁-C₄)alkylthio, (C₁-C₄)haloalkylthio, mono(C₁-C₄)alkylamino and di(C₁-C₄)alkylamino, or phenyl or (C₃-C₆)cycloalkyl, each of the last-mentioned 2 radicals being unsubstituted or substituted by one or more radicals selected from the group consisting of halogen, hydroxyl, amino, nitro, formyl, carboxyl, sulfonyl, cyano, thiocyanato, (C₁-C₄)alkyl, (C₁-C₄)haloalkyl, (C₁-C₄)alkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₉)cycloalkyl, [(C₁-C₄)alkyl]carbonyl, [(C₁-C₄)alkoxy]carbonyl, aminocarbonyl, mono(C₁-C₄)alkylamino-carbonyl, di(C₁-C₄)alkylaminocarbonyl, (C₁-C₄)alkylsulfonyl and (C₁-C₄)haloalkylsulfonyl, or a radical of the formula N(B¹-D¹)(B²-D²),
R⁴ is a radical of the formula -B³-D³, B³ and D³ being as defined below,
B¹, B² and B³ independently of one another are a direct bond or a divalent group of the formulae -C(=Z*)-, -C(=Z*)-Z**-, -C(=Z*)-NH- or -C(=Z*)-NR*-, where Z* = O or S, Z** = O or S and R* = (C₁-C₄)alkyl, phenyl, phenyl-(C₁-C₄)alkyl, (C₃-C₆)cycloalkyl or (C₃-C₆)cycloalkyl-(C₁-C₄)alkyl, each of the 5 last-mentioned radicals being unsubstituted or substituted by one or more radicals selected from the group consisting of halogen, hydroxyl, amino, nitro, formyl, carboxyl, sulfo, cyano, thiocyanato, (C₁-C₄)alkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₉)cycloalkyl, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, (C₁-C₄)alkylsulfonyl, (C₁-C₄)haloalkylsulfonyl and in the case of cyclic radicals also (C₁-C₄)alkyl and (C₁-C₄)haloalkyl;
D¹, D² and D³ independently of one another are hydrogen, (C₁-C₆)alkyl, phenyl, phenyl-(C₁-C₄)alkyl, (C₃-C₆)cycloalkyl or (C₃-C₆)cycloalkyl-(C₁-C₆)alkyl, each of the 5 last-mentioned radicals being unsubstituted or substituted by one or more radicals selected from the group consisting of halogen, hydroxyl, amino, nitro, formyl, carboxyl, sulfo, cyano, thiocyanato, (C₁-C₄)alkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₉)cycloalkyl, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, (C₁-C₄)alkylsulfonyl, (C₁-C₄)haloalkylsulfonyl and in the case of cyclic radicals also (C₁-C₄)alkyl and (C₁-C₄)haloalkyl,
(X)ₙ is n substituents X, where the X independently of one another are halogen, hydroxyl, amino, nitro, formyl, carboxyl, cyano, thiocyanato, aminocarbonyl or (C₁-C₄)alkyl, (C₁-C₄)alkoxy, (C₁-C₄)alkylthio, mono(C₁-C₄)alkylamino, di(C₁-C₄)alkylamino, (C₂-C₄)alkenyl, (C₂-C₄)alkynyl, [(C₁-C₄)alkyl]carbonyl, [(C₁-C₄)alkoxy]carbonyl, mono(C₁-C₄)alkylaminocarbonyl, di(C₁-C₄)alkylaminocarbonyl, N-(C₁-C₆)alkanoylamino or N-(C₁-C₄)alkanoyl-N-(C₁-C₄)alkylamino,
each of the last-mentioned 13 radicals being unsubstituted or substituted by one or more radicals selected from the group consisting of halogen, hydroxyl, amino, cyano, thiocyanato, (C₁-C₄)alkoxy, (C₁-C₄)haloalkoxy, (C₁-C₄)alkylthio, mono(C₁-C₄)alkylamino, di(C₁-C₄)alkylamino, (C₃-C₆)cycloalkyl, (C₃-C₆)cycloalkylamino, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, phenyl, phenoxy, phenylthio, phenylcarbonyl, heterocyclyl, heterocyclyloxy, heterocyclylthio and heterocyclylamino,
each of the last-mentioned 8 radicals being unsubstituted or having one or more substituents selected from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkylcarbonyl and (C₁-C₄)alkoxycarbonyl,
or (C₃-C₉)cycloalkyl, phenyl, phenoxy, phenylthio, phenylcarbonyl, heterocyclyl, heterocyclyloxy, heterocyclylthio or heterocyclylamino,
each of the last-mentioned 9 radicals being unsubstituted or substituted by one or more radicals selected from the group consisting of halogen, hydroxyl, amino, nitro, formyl, carboxyl, cyano, thiocyanato, (C₁-C₄)alkyl, (C₁-C₄)haloalkyl, (C₁-C₄)alkoxy, (C₁-C₄)haloalkoxy, (C₁-C₄)alkylthio, (C₁-C₄)haloalkylthio, mono(C₁-C₄)alkylamino, di(C₁-C₄)alkylamino, (C₃-C₆)cycloalkyl, [(C₁-C₄)alkyl]carbonyl, [(C₁-C₄)alkoxy]carbonyl, aminocarbonyl, mono(C₁-C₄)alkylaminocarbonyl and di(C₁-C₄)alkylaminocarbonyl,
or two adjacent radicals X together are a fused cycle which has 4 to 6 ring atoms and is carbocyclic or contains hetero ring atoms selected from the group consisting of O, S and N and which is unsubstituted or substituted by one or more radicals selected from the group consisting of halogen, (C₁-C₄)alkyl and oxo, and
n is 0, 1, 2 or 3 and
heterocyclyl in the abovementioned radicals independently of one another is in each case a heterocyclic radical having 3 to 7 ring atoms and 1 to 3 heteroatoms selected from the group consisting of N, O and S.

2. A compound of the formula (I) or a salt thereof as claimed in claim 1, wherein
R¹ is (C₁-C₄)alkyl which is unsubstituted or substituted by one or more radicals selected from the group consisting of halogen, (C₁-C₄)alkoxy, (C₁-C₄)alkylthio, (C₁-C₄)alkylsulfonyl, (C₃-C₆)cycloalkyl which is unsubstituted and phenyl which is unsubstituted or substituted by one or more radicals selected from the group consisting of halogen, (C₁-C₄)alkyl and (C₁-C₄)haloalkyl, (C₁-C₄)alkoxy, (C₁-C₄)haloalkoxy, (C₁-C₄)alkylthio, amino, mono- and di[(C₁-C₄)alkyl]amino, (C₁-C₄)alkanoylamino, benzoylamino, nitro, cyano, [(C₁-C₄)alkyl]carbonyl, formyl, carbamoyl, mono- and di-[(C₁-C₄)alkyl]aminocarbonyl and (C₁-C₄)alkylsulfonyl, and heterocyclyl having 3 to 6 ring atoms and 1 to 3 hetero ring atoms selected from the group consisting of N, O and S, the ring being unsubstituted or substituted by one or more radicals selected from the group consisting of halogen, (C₁-C₄)alkyl and oxo and, including substituents, has 2 to 30 carbon atoms.

3. A compound of the formula (I) or a salt thereof as claimed in claim 1 or 2, wherein
A¹ is straight-chain alkylene of the formula CH₂CH₂ and
A²-R² is cyclopropylmethyl, cyclobutyl or cyclobutylmethyl.

4. A compound of the formula (I) or a salt thereof as claimed in claim 1 or 2, wherein
A¹ is straight-chain alkylene of the formula CH₂CH₂CH₂ and
A²-R² is cyclopropyl, cyclopropylmethyl or cyclobutyl, cyclobutylmethyl.

5. A compound of the formula (I) or a salt thereof as claimed in any of claims 1 to 4, wherein
B¹, B² group and B³ independently of one another are a direct bond or a divalent of the formulae -C(=Z*)-, -C(=Z*)-Z**-, -C(=Z*)-NH- or -C(=Z*)-NR*- where Z* = O or S, Z** = O or S and R* = (C₁-C₄)alkyl, phenyl, phenyl-(C₁-C₄)alkyl, (C₃-C₆)cycloalkyl or (C₃-C₆)cycloalkyl(C₁-C₄)alkyl, each of the last-mentioned 5 radicals being unsubstituted or substituted by one or more radicals selected from the group consisting of halogen, hydroxyl, amino, formyl, (C₁-C₄)alkoxy, (C₁-C₄)haloalkoxy, (C₁-C₄)alkylthio, mono(C₁-C₄)alkylamino, di(C₁-C₄)alkylamino, (C₃-C₉)cycloalkyl, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl and in the case of cyclic radicals also (C₁-C₄)alkyl and (C₁-C₄)haloalkyl, and
D¹, D² and D³ independently of one another are (C₁-C₄)alkyl, (C₃-C₆)cycloalkyl, phenyl or phenyl-(C₁-C₄)alkyl, each of the last-mentioned two radicals being unsubstituted in the phenyl moiety or substituted in the phenyl moiety by one or more radicals selected from the group consisting of halogen, (C₁-C₄)alkyl, (C₁-C₄)haloalkyl, (C₁-C₄)alkoxy and (C₁-C₄)haloalkoxy.

6. A compound of the formula (I) or a salt thereof as claimed in any of claims 1 to 5, wherein
(X)ₙ is n substituents X, where X in each case independently of one another are halogen, OH, NO₂, CN, SCN(C₁-C₆)alkyl, (C₁-C₆)alkoxy, (C₁-C₄)alkylcarbonyl or (C₁-C₄)alkyloxycarbonyl, the last-mentioned four radicals being unsubstituted or substituted by halogen or (C₁-C₄)alkoxy.

7. A process for the preparation of a compound of the formula (I) or a salt thereof as claimed in any of claims 1 to 6, which comprises
a) reacting a compound of the formula (II)
R¹ - Fu (II)
where Fu is a functional group selected from the group consisting of carboxylic ester, carboxylic orthoester, carboxylic acid chloride, carboxamide, carboxylic anhydride and trichloromethyl with a biguanidide of the formula (III) or an acid addition salt thereof 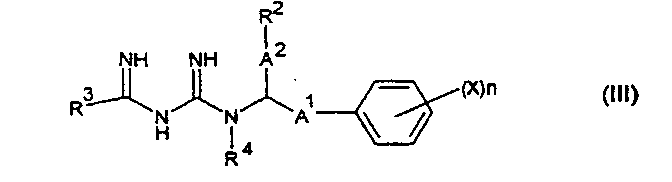 or
b) reacting a compound of the formula (IV) 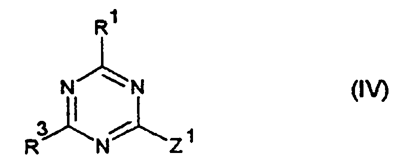
where Z¹ is an exchangeable radical or leaving group with a suitable amine of the formula (V) or an acid addition salt thereof 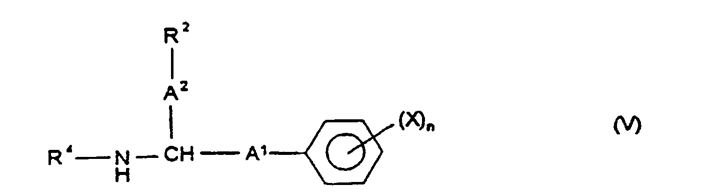 where, in the formulae (II), (III), (IV) and (V), the radicals R¹, R², R³, R⁴, A¹, A² and X and n are as defined in formula (I).

8. A herbicidal or plant-growth-regulating composition, which comprises one or more compounds of the formula (I) or salts thereof as claimed in any of claims 1 to 6 and formulation auxiliaries conventionally used in crop protection.

9. A method of controlling harmful plants or of regulating the growth of plants, wherein an effective amount of one or more compounds of the formula (I) or salts thereof as claimed in any of claims 1 to 6 is applied to the plants, the seeds of the plants or the area under cultivation.

10. The use of a compound of the formula (I) or a salt thereof as claimed in any of claims 1 to 6 as herbicides or plant growth regulators.

11. The use as claimed in claim 10, wherein the compounds of the formula (I) or the salts thereof are employed for controlling harmful plants or for regulating the growth in crops of useful plants or ornamentals.

12. The use as claimed in claim 11, wherein the crop plants are transgenic crop plants.

13. A compound of the formula (III) or (V) as defined in claim 7.

## Revendications

1. Composés de formule (I) ou leurs sels 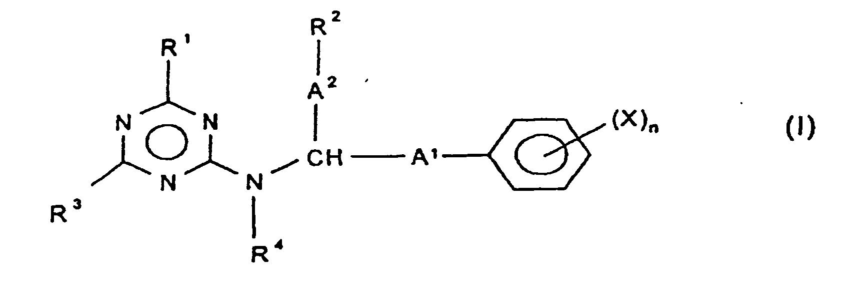 dans laquelle
R¹ représente un groupe phényle, qui est non substitué ou substitué par un ou plusieurs restes pris dans le groupe comprenant halogène, hydroxy, amino, nitro, formyle, carboxy, sulfo, cyano, thiocyanato, alkyle en C₁-C₄, haloalkyle en C₁-C₄, alkoxy en C₁-C₄, haloalkoxy en C₁-C₄, (alkyl en C₁-C₄)thio, halo(alkyl en C₁-C₄)thio, mono(alkyl en C₁-C₄)amino, di(alkyl en C₁-C₄)amino, cycloalkyle en C₃-C₉, (alkyl en C₁-C₄)carbonyle, (alkoxy en C₁-C₄)carbonyle, aminocarbonyle, mono(alkyl en C₁-C₄)amino-carbonyle, di(alkyl en C₁-C₄)amino-carbonyle, (alkyl en C₁-C₄)-sulfonyle et halo(alkyl en C₁-C₄)sulfonyle,
ou
un groupe cycloalkyle en C₃-C₉, qui est non substitué ou substitué par un ou plusieurs restes pris dans le groupe comprenant halogène, hydroxy, amino, cyano, thiocyanato, alkyle en C₁-C₄, haloalkyle en C₁-C₄, alkoxy en C₁-C₄, haloalkoxy en C₁-C₄, (alkyl en C₁-C₄)thio, halo(alkyl en C₁-C₄)thio, mono(alkyl en C₁-C₄)amino et di(alkyl en C₁-C₄)amino,
ou
hétérocyclyle, qui est non substitué ou substitué par un ou plusieurs restes pris dans le groupe comprenant halogène, hydroxy, amino, nitro, formyle, carboxy, sulfonyle, cyano, thiocyanato, alkyle en C₁-C₄, haloalkyle en C₁-C₄, alkoxy en C₁-C₄, haloalkoxy en C₁-C₄, (alkyl en C₁-C₄)thio, halo(alkyl en C₁-C₄)thio, mono(alkyl en C₁-C₄)amino, di(alkyl en C₁-C₄)amino, cycloalkyle en C₃-C₉, (alkyl en C₁-C₄)carbonyle, (alkoxy en C₁-C₄)carbonyle, aminocarbonyle, mono(alkyl en C₁-C₄)amino-carbonyle, di(alkyl en C₁-C₄)amino-carbonyle, (alkyl en C₁-C₄)-sulfonyle et halo(alkyl en C₁-C₄)sulfonyle,
ou
alkyle en C₁-C₄, alcényle en C₂-C₆ ou alcynyle en C₂-C₆,
chacun des trois derniers restes cités est non substitué ou substitué par un ou plusieurs restes pris dans le groupe comprenant halogène, hydroxy, cyano, nitro, thiocyanato, alkoxy en C₁-C₄, haloalkoxy en C₁-C₄, alcényloxy en C₂-C₄, haloalcényloxy en C₂-C₄, (alkyl en C₁-C₄)thio, (alkyl en C₁-C₄)sulfinyle, (alkyl en C₁-C₄)sulfonyle, halo(alkyl en C₁-C₄)sulfinyle, halo(alkyl en C₁-C₄)sulfonyle et cycloalkyle en C₃-C₆, qui est non substitué ou substitué par un ou plusieurs restes pris dans le groupe comprenant halogène, hydroxy, amino, cyano, thiocyanato, alkyle en C₁-C₄, haloalkyle en C₁-C₄, alkoxy en C₁-C₄, haloalkoxy en C₁-C₄, (alkyl en C₁-C₄)thio, halo(alkyl en C₁-C₄)thio, mono-(alkyl en C₁-C₄)amino et di(alkyl en C₁-C₄)amino, et phényle et hétérocyclyle, chacun des deux derniers restes cités est non substitué ou substitué par un ou plusieurs restes pris dans le groupe comprenant un groupe halogène, hydroxy, amino, nitro, formyle, carboxy,
sulfonyle, cyano, thiocyanato, alkyle en C₁-C₄, haloalkyle en C₁-C₄, alkoxy en C₁-C₄, haloalkoxy en C₁-C₄, (alkyl en C₁-C₄)thio, halo(alkyl en C₁-C₄)-thio, mono(alkyl en C₁-C₄)amino, di(alkyl en C₁-C₄)amino, cycloalkyle en C₃-C₉, (alkyl en C₁-C₄)-carbonyle, (alkoxy en C₁-C₄)carbonyle, aminocarbonyle, mono(alkyl en C₁-C₄)aminocarbonyle, di(alkyl en C₁-C₄)amino-carbonyle, (alkyl en C₁-C₄)sulfonyle et halo(alkyl en C₁-C₄)-sulfonyle, et des restes de formules R'-C(=Z')-, R'-C(=Z')-Z-, R'-Z-C(=Z')-, R'R"N-C(=Z')-, R'-Z-C(=Z')-O-, R'R"N-C(=Z')-Z-, R'-Z-C(=Z')-NR"- et R'R"N-C(=Z')-NR'"-,
R', R" et R"' chacun indépendamment l'un de l'autre représente un groupe alkyle en C₁-C₄, phényle, phénylalkyle en C₁-C₄, cycloalkyle en C₃-C₆ ou (cycloalkyl en C₃-C₆)-alkyle en C₁-C₄, chacun des 5 derniers restes cités est non substitué ou substitué par un ou plusieurs restes pris dans le groupe comprenant halogène, hydroxy, amino, nitro, formyle, cyano, thiocyanato, alkoxy en C₁-C₄, (alkyl en C₁-C₄)-thio, mono (alkyl en C₁-C₄)amino, di(alkyl en C₁-C₄)amino, alcényle en C₂-C₄, alcynyle en C₂-C₄, cycloalkyle en C₃-C₆ et, dans le cas de restes cycliques, aussi alkyle en C₁-C₄ et haloalkyle en C₁-C₄, et où Z et Z' chacun indépendamment l'un de l'autre représente un atome d'oxygène ou un atome de soufre,
A¹ représente un groupe alkylène linéaire de formule CH₂CH₂ et
A²-R² représente un groupe cyclopropyl-méthyle, 2-cyclopropyl-éthyle, cyclobutyle, cyclobutylméthyle ou 2-cyclobutyl-éthyle, ou
A¹ représente un groupe alkylène linéaire de formule CH₂CH₂CH₂ et
A²-R² représente un groupe cyclopropyle, cyclopropylméthyle, 2-cyclopropyl-éthyle, cyclobutyle, cyclobutyl-méthyle ou 2-cyclobutyl-éthyle, et
R³ représente un atome d'hydrogène, un groupe alkyle en C₁-C₄, qui est non substitué ou substitué par un ou plusieurs restes pris dans le groupe comprenant halogène, hydroxy, amino, cyano, thiocyanato, alkoxy en C₁-C₄, haloalkoxy en C₁-C₄, (alkyl en C₁-C₄)thio, halo(alkyl en C₁-C₄)-thio, mono(alkyl en C₁-C₄)amino et di(alkyl en C₁-C₄)amino, ou phényle ou cycloalkyle en C₃-C₆, chacun des 2 derniers restes cités est non substitué ou substitué par un ou plusieurs restes pris dans le groupe comprenant halogène, hydroxy, amino, nitro, formyle, carboxy, sulfonyle, cyano, thiocyanato, alkyle en C₁-C₄, haloalkyle en C₁-C₄, alkoxy en C₁-C₄, haloalkoxy en C₁-C₄, (alkyl en C₁-C₄)thio, halo(alkyl en C₁-C₄)-thio, mono(alkyl en C₁-C₄) amino, di (alkyl en C₁-C₄)amino, cycloalkyle en C₃-C₉, (alkyl en C₁-C₄)carbonyle, (alkoxy en C₁-C₄)carbonyle, aminocarbonyle, mono(alkyl en C₁-C₄)aminocarbonyle, di(alkyl en C₁-C₄)amino-carbonyle, (alkyl en C₁-C₄)sulfonyle et halo(alkyl en C₁-C₄)-sulfonyle, ou un reste de formule N(B¹-D¹) (B²-D²),
R⁴ représente un reste de formule -B³-D³, B³ et D³ définis comme ci-dessous,
B¹, B² et B³, indépendamment l'un de l'autre représentent une liaison directe ou un groupe bivalent de formules -C(=Z*)-, -C(=Z*)-Z**-, -C(=Z*)-NH- ou -C(=Z*)-NR*-, où Z* = O ou S, Z** = O ou S et R* = alkyle en C₁-C₄, phényle, phénylalkyle en C₁-C₄, cycloalkyle en C₃-C₆ ou (cycloalkyl en C₃-C₆)-alkyle en C₁-C₄, chacun des 5 derniers restes cités est non substitué ou substitué par un ou plusieurs restes pris dans le groupe comprenant halogène, hydroxy, amino, nitro, formyle, carboxy, sulfo, cyano, thiocyanato, alkoxy en C₁-C₄, haloalkoxy en C₁-C₄, (alkyl en C₁-C₄)thio, halo(alkyl en C₁-C₄)-thio, mono (alkyl en C₁-C₄)amino, di (alkyl en C₁-C₄)-amino, cycloalkyle en C₃-C₉, (alkyl en C₁-C₄)-carbonyle, (alkoxy en C₁-C₄)carbonyle, aminocarbonyle, mono(alkyl en C₁-C₄)aminocarbonyle, di(alkyl en C₁-C₄)amino-carbonyle, (alkyl en C₁-C₄)sulfonyle, halo(alkyl en C₁-C₄)sulfonyle et, dans le cas de restes cycliques, aussi alkyle en C₁-C₄ et haloalkyle en C₁-C₄ ;
D¹, D² et D³ indépendamment les uns des autres représentent
un atome d'hydrogène, un groupe alkyle en C₁-C₆, phényle, phénylalkyle en C₁-C₄, cycloalkyle en C₃-C₆ ou (cycloalkyl en C₃-C₆)-alkyle en C₁-C₆, chacun des 5 derniers restes cités est non substitué ou substitué par un ou plusieurs restes pris dans le groupe comprenant halogène, hydroxy, amino, nitro, formyle, carboxy, sulfo, cyano, thiocyanato, alkoxy en C₁-C₄, haloalkoxy en C₁-C₄, (alkyl en C₁-C₄)thio, (haloalkyl en C₁-C₄)-thio, mono(alkyl en C₁-C₄)amino, di(alkyl en C₁-C₄)amino, cycloalkyle en C₃-C₉, (alkyl en C₁-C₄)-carbonyle, (alkoxy en C₁-C₄)carbonyle, aminocarbonyle, mono(alkyl en C₁-C₄)aminocarbonyle, di (alkyl en C₁-C₄)amino-carbonyle, (alkyl en C₁-C₄)sulfonyle, (haloalkyl en C₁-C₄)sulfonyle et, dans le cas de restes cycliques, aussi alkyle en C₁-C₄ et haloalkyle en C₁-C₄,
(X)ₙ représente n substituants X et les substituants X indépendamment les uns des autres représentent un atome d'halogène, un groupe hydroxy, amino, nitro, formyle, carboxy, cyano, thiocyanato, aminocarbonyle ou alkyle en C₁-C₄, alkoxy en C₁-C₄, (alkyl en C₁-C₄)thio, mono(alkyl en C₁-C₄)amino, di(alkyl en C₁-C₄)amino, alcényle en C₂-C₄, alcynyle en C₂-C₄, (alkyl en C₁-C₄)carbonyle, (alkoxy en C₁-C₄)carbonyle, mono(alkyl en C₁-C₄)amino-carbonyle, di(alkyl en C₁-C₄)-amino-carbonyle, N- (alcanoyl en C₁-C₆)amino ou N-(alcanoyl en C₁-C₄)-N- (alkyl en C₁-C₄)-amino,
chacun des 13 derniers restes cités est non substitué ou substitué par un ou plusieurs restes pris dans le groupe comprenant halogène, hydroxy, amino, cyano, thiocyanato, alkoxy en C₁-C₄, haloalkoxy en C₁-C₄, (alkyl en C₁-C₄)thio, mono(alkyl en C₁-C₄)amino, di(alkyl en C₁-C₄)-amino, cycloalkyle en C₃-C₆, (cycloalkyl en C₃-C₆)amino, (alkyl en C₁-C₄)-carbonyle, (alkoxy en C₁-C₄)carbonyle, aminocarbonyle, mono(alkyl en C₁-C₄) amino-carbonyle, di (alkyl en C₁-C₄)amino-carbonyle, phényle, phénoxy, phénylthio, phénylcarbonyle, hétérocyclyle, hétérocyclyloxy, hétérocyclylthio et hétérocyclylamino, chacun des 8 derniers restes cités est non substitué ou présente un ou plusieurs substituants pris dans le groupe comprenant halogène, nitro, cyano, alkyle en C₁-C₄, alkoxy en C₁-C₄, (alkyl en C₁-C₄)-thio, haloalkyle en C₁-C₄, haloalkoxy en C₁-C₄, formyle, (alkyl en C₁-C₄)-carbonyle et (alkoxy en C₁-C₄)carbonyle,
ou cycloalkyle en C₃-C₉, phényle, phénoxy, phénylthio, phénylcarbonyle, hétérocyclyle, hétérocyclyloxy, hétérocyclylthio ou hétérocyclylamino, chacun des 9 derniers restes cités est non substitué ou substitué par un ou plusieurs restes pris dans le groupe comprenant halogène, hydroxy, amino, nitro, formyle, carboxy, cyano, thiocyanato, alkyle en C₁-C₄, haloalkyle en C₁-C₄, alkoxy en C₁-C₄, haloalkoxy en C₁-C₄, (alkyl en C₁-C₄)thio, halo(alkyl en C₁-C₄)thio, mono(alkyl en C₁-C₄)amino, di(alkyl en C₁-C₄)amino, cycloalkyle en C₃-C₆, (alkyl en C₁-C₄)carbonyle, (alkoxy en C₁-C₄)carbonyle, aminocarbonyle, mono(alkyl en C₁-C₄)aminocarbonyle et di(alkyl en C₁-C₄)aminocarbonyle,
ou deux restes X voisins forment conjointement un cycle condensé présentant 4 à 6 chaînons, qui est carbocyclique ou présente des hétéroatomes pris dans le groupe comprenant un atome de O, de S et de N, et qui est non substitué ou substitué par un ou plusieurs restes pris dans le groupe comprenant halogène, alkyle en C₁-C₄ et oxo, et
n vaut 0, 1, 2 ou 3
et
les groupes hétérocyclyle dans les restes précités présentent indépendamment les uns des autres un reste hétérocyclique présentant 3 à 7 chaînons et 1 à 3 hétéroatomes pris dans le groupe comprenant un atome de N, de O et de S.

2. Composé de formule (I) et ses sels selon la revendication 1, **caractérisé en ce que**
R¹ représente un groupe alkyle en C₁-C₄, qui est non substitué ou substitué par un ou plusieurs restes pris dans le groupe comprenant halogène, alkoxy en C₁-C₄, (alkyl en C₁-C₄)thio, (alkyl en C₁-C₄)sulfonyle, cycloalkyle en C₃-C₆, qui est non substitué, et phényle, qui est non substitué ou substitué par un ou plusieurs restes pris dans le groupe comprenant halogène, alkyle en C₁-C₄ et haloalkyle en C₁-C₄, alkoxy en C₁-C₄, haloalkoxy en C₁-C₄, (alkyl en C₁-C₄)thio, amino, mono- et di(alkyl en C₁-C₄)amino, (alcanoyl en C₁-C₄)amino, benzoylamino, nitro, cyano, (alkyl en C₁-C₄)carbonyle, formyle, carbamoyle, mono- et di-(alkyl en C₁-C₄)aminocarbonyle et (alkyl en C₁-C₄)sulfonyle, et hétérocyclyle présentant 3 à 6 chaînons et 1 à 3 hétéroatomes pris dans le groupe comprenant un atome de N, de O et de S, le cycle étant non substitué ou substitué par un ou plusieurs restes pris dans le groupe comprenant halogène, alkyle en C₁-C₄ et oxo et présente, y compris les substituants, 2 à 30 atomes de carbone.

3. Composé de formule (I) et ses sels selon la revendication 1 ou 2, **caractérisé en ce que**
A¹ représente un groupe alkylène linéaire de formule CH₂CH₂ et
A²-R² représente un groupe cyclopropyl-méthyle, cyclobutyle ou cyclobutyl-méthyle.

4. Composé de formule (I) et ses sels selon la revendication 1 ou 2, **caractérisé en ce que**
A¹ représente un groupe alkylène linéaire de formule CH₂CH₂CH₂ et
A²-R² représente un groupe cyclopropyle, cyclopropylméthyle, cyclobutyle ou cyclobutyl-méthyle.

5. Composé de formule (I) et ses sels selon l'une des revendications 1 à 4, **caractérisé en ce que**
B¹, B² et B³, indépendamment les uns des autres représentent une liaison directe ou un groupe bivalent de formules -C(=Z*)-, -C(=Z*)-Z**-, -C(=Z*)-NH- ou -C(=Z*)-NR*-, où Z* = 0 ou S, Z** = O ou S et R* = alkyle en C₁-C₄, phényle, phénylalkyle en C₁-C₄, cycloalkyle en C₃-C₆ ou (cycloalkyl en C₃-C₆)-alkyle en C₁-C₄, chacun des 5 derniers restes cités est non substitué ou substitué par un ou plusieurs restes pris dans le groupe comprenant halogène, hydroxy, amino, formyle, alkoxy en C₁-C₄, haloalkoxy en C₁-C₄, (alkyl en C₁-C₄)thio, mono(alkyl en C₁-C₄)amino, di(alkyl en C₁-C₄)amino, cycloalkyle en C₃-C₉, (alkyl en C₁-C₄)carbonyle, (alkoxy en C₁-C₄)carbonyle, aminocarbonyle, mono(alkyl en C₁-C₄)amino-carbonyle, di(alkyl en C₁-C₄)aminocarbonyle et, dans le cas de restes cycliques, aussi alkyle en C₁-C₄ et haloalkyle en C₁-C₄, et
D¹,D² et D³ indépendamment les uns des autres représentent un groupe alkyle en C₁-C₄, cycloalkyle en C₃-C₆, phényle ou phénylalkyle en C₁-C₄, chacun des deux derniers restes cités est non substitué dans le fragment phényle ou substitué par un ou plusieurs restes pris dans le groupe comprenant halogène, alkyle en C₁-C₄, haloalkyle en C₁-C₄, alkoxy en C₁-C₄ et haloalkoxy en C₁-C₄.

6. Composé de formule (I) et ses sels selon l'une des revendications 1 à 5, **caractérisé en ce que**
(X)ₙ représente n substituants X et les substituants X à chaque fois indépendamment les uns des autres représentent un atome d'halogène, un groupe OH, NO₂, CN, SCN alkyle en C₁-C₆, alkoxy en C₁-C₆, (alkyl en C₁-C₄)carbonyle ou (alkyl en C₁-C₄)oxycarbonyle, les quatre derniers restes cités sont non substitués ou substitués par un substituant halogène ou alkoxy en C₁-C₄.

7. Procédé pour la préparation des composés de formule générale (I) ou de leurs sels selon l'une des revendications 1 à 6, **caractérisé, en ce qu'**on fait réagir
a) un composé de formule (II),
**R**^{**1**} **- Fu** (II)
où Fu est un groupe fonctionnel pris dans le groupe comprenant des esters d'acides carboxyliques, des orthoesters d'acides carboxyliques, des chlorures d'acides carboxyliques, des amides d'acides carboxyliques, des anhydrides d'acides carboxyliques et trichlorométhyle, sur un biguanidide de formule (III) ou un de ses sels d'addition d'acide 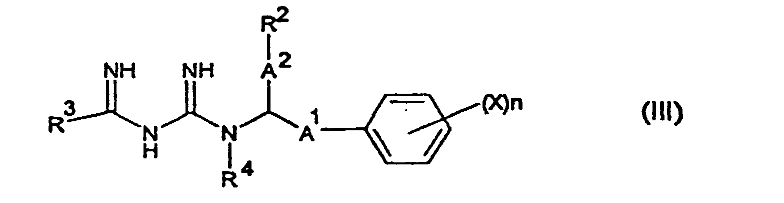 ou
b) un composé de formule (IV), 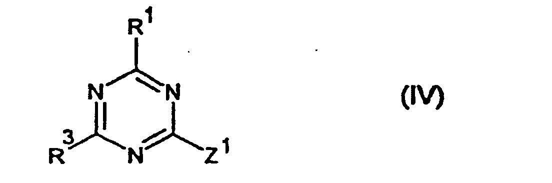
où Z¹ représente un reste échangeable ou un groupe partant, sur une amine appropriée de formule (V) ou un sel d'addition d'acide de celle-ci, 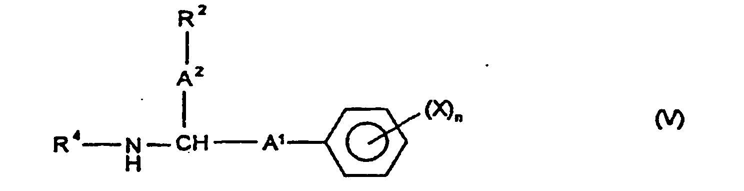 dans les formules (II), (III), (IV) et (V), les restes R¹, R², R³, R⁴, A¹, A² et X ainsi que n sont définis comme à la formule (I).

8. Agent herbicide ou régulateur de croissance de plantes **caractérisé en ce qu'**il renferme un ou plusieurs composés de formule (I) ou leurs sels selon l'une des revendications 1 à 6 et des formulants usuels dans le domaine phytoprotecteur.

9. Procédé pour la lutte contre les mauvaises herbes ou pour la régulation de la croissance de plantes, **caractérisé en ce qu'**on applique une quantité efficace d'un ou plusieurs composés de formule (I) ou de leurs sels selon l'une des revendications 1 à 6, aux plantes, à la semence de plantes ou à la surface cultivable.

10. Utilisation de composés de formule (I) ou de leurs sels selon l'une des revendications 1 à 6, en tant qu'herbicides ou régulateurs de croissance végétale.

11. Utilisation selon la revendication 10, **caractérisée en ce qu'**on utilise les composés de formule (I) ou leurs sels pour la lutte contre les plantes adventices ou pour la régulation de la croissance dans les cultures de plantes utiles ou décoratives.

12. Utilisation selon la revendication 11, **caractérisée en ce que** les plantes de culture sont des plantes de culture transgéniques.

13. Composés de formule (III) ou (V) tels que définis selon la revendication 7.
